(19) Europäisches Patentamt / European Patent Office / Office européen des brevets

(11) **EP 3 432 254 A1**

(12) **EUROPEAN PATENT APPLICATION**
published in accordance with Art. 153(4) EPC

(43) Date of publication:
**23.01.2019 Bulletin 2019/04**

(21) Application number: **17766820.9**

(22) Date of filing: **16.03.2017**

(51) Int Cl.:
*G06Q 50/22* (2018.01)    *G06Q 40/08* (2012.01)

(86) International application number:
**PCT/JP2017/010791**

(87) International publication number:
**WO 2017/159825 (21.09.2017 Gazette 2017/38)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **16.03.2016   JP 2016053159**
**18.08.2016   JP 2016160910**
**18.08.2016   JP 2016160911**
**18.08.2016   JP 2016160912**

(71) Applicant: **Medcare, Inc.**
**Tokyo 163-1030 (JP)**

(72) Inventor: **AKASHI, Hideyuki**
**Tokyo 163-1030 (JP)**

(74) Representative: **Weickmann & Weickmann PartmbB**
**Postfach 860 820**
**81635 München (DE)**

(54) **REMOTE DIAGNOSTIC AID SYSTEM, REMOTE DIAGNOSTIC AID SERVER, AND REMOTE DIAGNOSTIC AID METHOD**

(57)    A remote diagnostic aid system capable of appropriately determining the amount paid by an organization that bears the whole or part of medical expenses for remote diagnosis by self-funded care is provided. The system includes a medical expense determination unit which determines medical expenses for an instant examinee, based on medical record information, such that the medical expenses become lower than standard medical expenses for the same content of diagnosis in the case of face-to-face diagnosis, and an organization's payment determination unit which determines, as a system utilization cost, a portion of the difference between an organization's standard payment of medical expenses, which is obtained by multiplying the standard medical expenses by an organization's share of medical expenses of an organization to which the instant examinee belongs, and an organization's payment of medical expenses, which is obtained by multiplying the medical expenses by the organization's share of medical expenses.

**FIG.7B**

```
REMOTE MEDICAL AID SERVER
                              STEP4011
RECOGNIZE MEDICAL REMUNERATION POINTS
                              STEP4012
RECOGNIZE MEDICAL EXPENSES IN
CASE OF FACE-TO-FACE DIAGNOSIS
                              STEP4013
RECOGNIZE MEDICAL EXPENSES FOR REMOTE DIAGNOSIS
                              STEP4014
RECOGNIZE POINTS THIS TIME AND CUMULATIVE POINTS
                              STEP4015
APPLY DISCOUNT ACCORDING TO THE POINTS

              END
```

**Description**

Technical Field

The present invention relates to a remote diagnostic aid system, a remote diagnostic aid server, and a remote diagnostic aid method.

Background Art

Remote diagnosis is conventionally known in which a doctor makes a diagnosis of an examinee via information communication technology (see, for example, Patent Literature 1).

In remote diagnosis, generally, accepting persons who want to have consultations, managing the persons waiting for consultations, and accounting operations are performed automatically to reduce labor costs. This allows a medical facility to provide remote diagnosis with reduced costs.

In particular, patients with chronic diseases need to receive medical care on a regular basis. Remote diagnosis will be optimal for patients who cannot make time for face-to-face diagnosis.

Citation List

Patent Literature

Patent Literature 1: Japanese Patent Application Laid-Open No. 2015-90572

Summary of Invention

Technical Problem

It is therefore expected that there is a potential demand for remote diagnosis particularly from busy patients with chronic diseases.

Although patients with chronic diseases need to receive medical care on a regular basis, if an examinee must pay a system utilization cost for the system providing the remote diagnosis or expensive medical fees, the amount paid by the examinee will increase, making it rather difficult for the examinee to utilize the system.

In view of the foregoing, an object of the present invention is to provide a remote diagnostic aid system, a remote diagnostic aid server, and a remote diagnostic aid method which enable reduction of the amount paid by the examinee.

Solution to Problem

A remote diagnostic aid system according to the present invention is
a network system including a remote diagnostic aid server and one or more doctor terminals which communicate with a plurality of user terminals, and the system includes:

> a storage unit which stores available consultation time information including an available consultation time during which a doctor as a user of a respective one of the one or more doctor terminals is available for consultation, and payment rate information indicating, for a respective one of users of the plurality of user terminals, a copayment rate which is a payment rate of the user and an organization's share of medical expenses which is a payment rate of an organization to which the user belongs;
> a consultation appointment acceptance unit which receives, from a first user terminal as one of the plurality of user terminals, information including a desired consultation time during which an instant examinee as a user of the first user terminal wants to have a consultation, refers to the available consultation time information to extract an attending doctor who is available for consultation during the desired consultation time, and transmits, to one or both of the first user terminal and an attending doctor terminal as the doctor terminal of the attending doctor, communication establishment information for establishing a communication between the attending doctor terminal and the first user terminal;
> a remote diagnostic aid unit which outputs examinee's statement information, received from the first user terminal with which the communication has been established on the basis of the communication establishment information, to the attending doctor terminal, and transmits doctor's statement information, input from the attending doctor terminal, to the first user terminal;

a medical expense determination unit which determines medical expenses for the instant examinee, on the basis of medical record information of the instant examinee received from the attending doctor terminal or the first user terminal, such that the medical expenses become lower than standard medical expenses for the same content of diagnosis in a case of face-to-face diagnosis; and

an organization's payment determination unit which determines, as a system utilization cost, a portion of a difference between an organization's standard payment of medical expenses, obtained by multiplying the standard medical expenses by the organization's share of medical expenses of an organization to which the instant examinee belongs, and an organization's payment of medical expenses, obtained by multiplying the medical expenses by the organization's share of medical expenses.

According to the remote diagnostic aid system with the above configuration, the consultation appointment acceptance unit receives information including a desired consultation time from the first user terminal, and refers to the available consultation time information to extract an attending doctor who is available for consultation during the desired consultation time.

An appointment for consultation is thus made within the time desired by the instant examinee and during which the attending doctor is available. Even if the instant examinee cannot make time for receiving face-to-face diagnosis, the examinee can be given a diagnosis by designating the time for consultation. This alleviates the time-wise cost of the instant examinee. In addition, the consultation can be held at a time convenient for the attending doctor. Work opportunities are thus offered to doctors who have difficulties in performing medical examinations full time, like those having children.

The communication establishment information for establishing a communication between the attending doctor terminal and the first user terminal is transmitted by the consultation appointment acceptance unit to one or both of the attending doctor terminal and the first user terminal.

The examinee's statement information, received from the first user terminal with which the communication has been established on the basis of the communication establishment information, is output by the remote diagnostic aid unit to allow the attending doctor to recognize the examinee's statement information.

The input doctor's statement information is transmitted by the remote diagnostic aid unit to the first user terminal. As this doctor's statement information is output at the first user terminal, the instant examinee can recognize the doctor's statement information input by the attending doctor.

In this manner, the remote diagnosis between the instant examinee and the attending doctor is aided.

Further, the medical expenses for the instant examinee are determined by the medical expense determination unit, on the basis of the received medical record information, such that the medical expenses become lower than standard medical expenses for the same content of diagnosis in the case of face-to-face diagnosis.

Further, a portion of the difference between the organization's standard payment of medical expenses, obtained by multiplying the standard medical expenses by the organization's share of medical expenses, and the organization's payment of medical expenses, obtained by multiplying the medical expenses by the organization's share of medical expenses, is determined as a system utilization cost by the organization's payment determination unit.

The medical expenses for the instant examinee are determined, as described above, to become lower than the standard medical expenses in the case of face-to-face diagnosis. Therefore, the organization's payment of medical expenses, which is an amount obtained by multiplying the medical expenses by the organization's share of medical expenses, becomes lower than the organization's standard payment of medical expenses. Since the system utilization cost is a portion of the difference between the organization's standard payment of medical expenses and the organization's payment of medical expenses, a sum of the organization's payment of medical expenses and the system utilization cost becomes lower than the organization's standard payment of medical expenses in the case of face-to-face diagnosis.

Accordingly, even if the organization pays the system utilization cost, the amount paid by the organization becomes smaller than the organization's standard payment of medical expenses in the case of face-to-face diagnosis. The payment by the organization is thus reduced as compared to the case of face-to-face diagnosis.

The copayment of medical expenses by the instant examinee is derived by deducting the organization's payment of medical expenses from the medical expenses for the instant examinee. The medical expenses for the instant examinee have been determined so as to be lower than the medical expenses for the same content of diagnosis in the case of face-to-face diagnosis. Accordingly, the copayment of medical expenses by the instant examinee also becomes lower than the copayment for the same content of diagnosis in the case of face-to-face diagnosis with the same organization's share. The payment by the instant examinee is thus also reduced.

In the remote diagnostic aid system of the present invention, it is preferable that

the storage unit stores information on an organization's payment of medical expenses in which a consultation time point, an examinee, and an organization's payment of medical expenses are associated with one another, and

the remote diagnostic aid system is configured to include a data transmission unit which refers to the information on the organization's payment of medical expenses to transmit, to a terminal of an organization, data on an organization's payment of medical expenses indicating a relationship between the consultation time point and the organization's payment of medical expenses for an examinee associated with the organization.

According to the remote diagnostic aid system with the above configuration, the data on the organization's payment of medical expenses indicating the relationship between the consultation time point and the organization's payment of medical expenses is transmitted to the terminal of the organization with which the examinee is enrolled.

Particularly in the case where a chronic disease of a member is healing or getting better, the medical expenses become lower gradually and, thus, the organization's payment of medical expenses also decreases gradually. The data on the organization's payment of medical expenses indicating such a transition of the organization's payment can let a person in charge in the organization recognize the time-series effect of reduction of the organization's payment of medical expenses through utilization of the remote diagnostic aid system.

In the remote diagnostic aid system with the above configuration, it is preferable that

the storage unit stores information on an organization's standard payment of medical expenses in which a consultation time point, an examinee, and an organization's standard payment of medical expenses are associated with one another, and

the remote diagnostic aid system is configured to include a data transmission unit which refers to the information on the organization's standard payment of medical expenses to transmit, to a terminal of an organization, data on an organization's standard payment of medical expenses indicating a relationship between the consultation time point and the organization's standard payment of medical expenses for an examinee associated with the organization, together with the data on the organization's payment of medical expenses.

According to the remote diagnostic aid system with the above configuration, the data on the organization's standard payment of medical expenses and the data on the organization's payment of medical expenses are transmitted to the organization terminal, to allow the person in charge to understand the transition of the organization's payment of medical expenses in comparison with the organization's standard payment of medical expenses. Particularly when the organization's payment of medical expenses is smaller than the organization's standard payment of medical expenses, the effect of reduction of the organization's payment of medical expenses can be made known to the person in charge more effectively.

In the remote diagnostic aid system of the present invention, it is preferable that

the storage unit stores medical record information in which a consultation time point, an examinee, and one or both of health condition information of the examinee and information on medicines prescribed for the examinee are associated with one another, and

the remote diagnostic aid system is configured to include a data transmission unit which refers to the medical record information to transmit, to a user terminal of an examinee, first health condition data associated with the examinee indicating a relationship between the consultation time point and one or both of the health condition information and the information on the medicines prescribed for the examinee.

According to the remote diagnostic aid system with the above configuration, the first health condition data indicating the relationship between the consultation time point and one or both of the examinee's health condition information and the information on the medicines prescribed for the examinee, included in the medical record information, is transmitted to the user terminal of the examinee. This allows the examinee to be notified of the time-series transitions of the examinee's health conditions by utilization of the remote diagnostic aid system.

In the case of a chronic disease requiring long-term therapy, even if a patient is getting better, it would be difficult for the patient to actually feel it. This leads to a drop in patient's motivation for continuous consultations, probably making the patient pull away from the consultations.

According to the remote diagnostic aid system with the above configuration, the first health condition data is provided to the user terminal. When the examinee's health conditions are improving through utilization of the remote diagnosis, the data allows the examinee to visually realize that the examinee is getting better. This may prevent the drop in examinee's motivation for continuous consultations; rather, it can encourage the examinee to continuously receive consultations in remote diagnosis.

In the remote diagnostic aid system of the present invention, it is preferable that

the storage unit stores medical record information in which a consultation time point, and one or both of health condition information and information on prescribed medicines including a disease name are associated with each other, and

the remote diagnostic aid system is configured to include a data transmission unit which

receives, from a second user terminal as one of the user terminals, a request for health condition information including a disease name, and refers to the medical record information to extract one or both of the health condition information and the information on the prescribed medicines including the received disease name, and

transmits, to the second user terminal, second health condition data indicating time-series transitions of one or both of the extracted health condition information and the extracted information on the prescribed medicines.

According to the remote diagnostic aid system with the above configuration, when a request for information on health conditions including a disease name is received from the second user terminal, the data transmission unit refers to the medical record information to extract one or both of the health condition information and the information on the prescribed medicines including the received disease name.

The data transmission unit then transmits to the second user terminal the second health condition data indicating time-series transitions of one or both of the extracted health condition information and the extracted information on the prescribed medicines.

Even for a user who has not utilized the remote diagnostic aid server before, when the request for health condition information including the disease name of interest is transmitted to the remote diagnostic aid server via the second user terminal, the second health condition data is transmitted from the remote diagnostic aid server.

In this manner, information useful for considering whether to utilize the remote diagnostic aid server can be provided even to a user who has not utilized the remote diagnostic aid server before.

A remote diagnostic aid system according to the present invention is

a network system including a remote diagnostic aid server and one or more doctor terminals communicating with a plurality of user terminals, and the remote diagnostic aid system includes:

a storage unit which stores available consultation time information including an available consultation time during which a doctor associated with a respective one of the one or more doctor terminals is available for consultation, and information on an insurance organization's standard payment which is a standard amount to be paid by an insurance organization;

a consultation appointment acceptance unit which receives, from a first user terminal as one of the plurality of user terminals, a desired consultation time during which an instant examinee associated with the first user terminal wants to have a consultation, refers to the available consultation time information to extract an attending doctor who is available for consultation during the desired consultation time, and transmits, to one or both of the first user terminal and an attending doctor terminal as the doctor terminal of the attending doctor, communication establishment information for establishing a communication between the attending doctor terminal and the first user terminal;

a remote diagnostic aid unit which outputs examinee's statement information, received from the first user terminal with which the communication has been established on the basis of the communication establishment information, to the attending doctor terminal, and transmits doctor's statement information, input from the attending doctor terminal, to the first user terminal; and

a system utilization cost settlement unit which, on the basis of medical record information of the instant examinee received from the attending doctor terminal or the first user terminal, calculates a part or whole of medical expenses for the instant examinee as an insurance organization's payment which is to be paid by an insurance organization with which the instant examinee is enrolled, and uses settlement information of the insurance organization to make settlement of a portion of a difference between the calculated insurance organization's payment and the insurance organization's standard payment as a system utilization cost.

According to the remote diagnostic aid system with the above configuration, the consultation appointment acceptance unit receives a desired consultation time from the first user terminal and refers to the available consultation time information to extract an attending doctor who is available for consultation in the desired consultation time.

With this, an appointment for consultation is made within the time desired by the instant examinee and during which the attending doctor is available. Even if the instant examinee cannot make time for receiving face-to-face diagnosis, the examinee can be given a diagnosis by designating the time for consultation. This alleviates the time-wise cost of the instant examinee. In addition, the consultation can be held at a time convenient for the attending doctor. Work opportunities are thus offered to doctors who have difficulties in performing medical examinations full time, like those having children.

The communication establishment information for establishing a communication between the attending doctor terminal and the first user terminal is transmitted by the consultation appointment acceptance unit to one or both of the attending doctor terminal and the first user terminal.

The examinee's statement information, received from the first user terminal with which the communication has been established on the basis of the communication establishment information, is output by the remote diagnostic aid unit to allow the attending doctor to recognize the examinee's statement information.

The input doctor's statement information is transmitted by the remote diagnostic aid unit to the first user terminal. As this doctor's statement information is output at the first user terminal, the instant examinee can recognize the doctor's statement information input by the attending doctor.

In this manner, the remote diagnosis between the instant examinee and the attending doctor is aided.

The insurance organization's payment, which is a part or whole of the medical expenses of the instant examinee, is calculated by the system utilization cost settlement unit, on the basis of the received medical record information.

Further, by the system utilization cost settlement unit, the insurance organization with which the instant examinee is enrolled is caused to make settlement of a portion of the difference between the insurance organization's payment and the insurance organization's standard payment as a system utilization cost, by using the settlement information of the insurance organization. With this, the system utilization cost is collected, not from the instant examinee, but from the insurance organization with which the instant examinee is enrolled.

This allows the instant examinee to utilize the remote diagnosis without paying the system utilization cost. This reduces the instant examinee's payment for a consultation of the remote diagnosis.

In addition, a portion of the difference between the insurance organization's payment and the insurance organization's standard payment is determined as the system utilization cost. Here, the insurance organization's payment, which is an insurance coverage of the medical expenses for the remote diagnosis, is lower than the insurance organization's payment in the case of face-to-face diagnosis, because the medical expenses for the remote diagnosis are made lower than the medical expenses in the case of face-to-face diagnosis as described above. Accordingly, as the insurance organization's payment is smaller than the insurance organization's standard payment, a sum of the insurance organization's payment and the system utilization cost as a portion of the difference between the insurance organization's payment and the insurance organization's standard payment also becomes smaller than the insurance organization's standard payment. Thus, there is substantially no increase in payment of the insurance organization. Rather, the insurance premium of the insurance organization may become lower, probably leading to a less economical cost for the examinee.

Further, as explained above, in the remote diagnostic aid system, work opportunities are offered to doctors who have difficulties in performing medical examinations full time, which facilitates securing doctors. In addition, the operational cost for the remote diagnostic aid system is covered by the system utilization cost collected from the insurance organization as explained above. These reduce the payment by the facility that offers the remote diagnosis.

As described above, according to the remote diagnostic aid system with the above configuration, it is possible to reduce both of the examinee's economical cost and the payment by the facility that offers the remote diagnosis.

A remote diagnostic aid system according to the present invention is a network system including a remote diagnostic aid server and one or more doctor terminals communicating with a plurality of user terminals, and the remote diagnostic aid system includes:

> a storage unit which stores available consultation time information including an available consultation time during which a doctor as a user of a respective one of the one or more doctor terminals is available for consultation;
> a consultation appointment acceptance unit which receives, from a first user terminal as one of the plurality of user terminals, information including a desired consultation time during which an instant examinee as a user of the first user terminal wants to have a consultation, refers to the available consultation time information to extract an attending doctor who is available for consultation during the desired consultation time, and transmits, to one or both of the first user terminal and an attending doctor terminal as the doctor terminal of the attending doctor, communication establishment information for establishing a communication between the attending doctor terminal and the first user terminal;
> a remote diagnostic aid unit which outputs examinee's statement information, received from the first user terminal with which the communication has been established on the basis of the communication establishment information, to the attending doctor terminal, and transmits doctor's statement information, input from the attending doctor terminal, to the first user terminal; and
> a medical expense determination unit which recognizes a condition of the instant examinee on the basis of medical record information received from the attending doctor terminal or the first user terminal, determines whether the condition of the instant examinee fulfills a current stipulation, determines medical expenses for the instant examinee such that the medical expenses when it is determined that the condition of the instant examinee fulfills the current stipulation become not higher than the medical expenses when it is determined that the condition of the instant examinee fails to fulfill the current stipulation, recognizes information indicating a next stipulation on the basis of the medical record information, and transmits the medical expenses, whether the current stipulation has been fulfilled or not, and the information indicating the next stipulation to the first user terminal.

According to the remote diagnostic aid system with the above configuration, the consultation appointment acceptance unit receives information including the desired consultation time from the first user terminal, and refers to the available consultation time information to extract an attending doctor who is available for consultation during the desired consultation time.

An appointment for consultation is thus made within the time desired by the instant examinee and during which the attending doctor is available. Even if the instant examinee cannot make time for receiving face-to-face diagnosis, the examinee can be given a diagnosis by designating the time for consultation. This alleviates the time-wise cost of the instant examinee. In addition, the consultation can be held at a time convenient for the attending doctor. Work opportunities are thus offered to doctors who have difficulties in performing medical examinations full time, like those having children.

The communication establishment information for establishing a communication between the attending doctor terminal and the first user terminal is transmitted by the consultation appointment acceptance unit to one or both of the attending doctor terminal and the first user terminal.

The examinee's statement information, received from the first user terminal with which the communication has been established on the basis of the communication establishment information, is output by the remote diagnostic aid unit, so that the attending doctor can recognize the examinee's statement information.

The input doctor's statement information is transmitted by the remote diagnostic aid unit to the first user terminal. As this doctor's statement information is output at the first user terminal, the instant examinee can recognize the doctor's statement information input by the attending doctor.

In this manner, the remote diagnosis between the instant examinee and the attending doctor is aided.

The instant examinee's condition is recognized on the basis of the medical record information received from the attending doctor terminal or the first user terminal. The medical expense determination unit determines whether the instant examinee's condition fulfills the current stipulation. The medical expenses of the instant examinee are determined by the medical expense determination unit such that the medical expenses when it is determined that the instant examinee's condition fulfills the current stipulation become not higher than the medical expenses when it is determined that the instant examinee's condition fails to fulfill the current stipulation.

In other words, the amount paid by the instant examinee when the instant examinee's condition fulfills the current stipulation becomes not greater than that when the instant examinee's condition fails to fulfill the current stipulation.

The information indicating a next stipulation is recognized by the medical expense determination unit on the basis of the medical record information, and the medical expenses, whether the current stipulation has been fulfilled or not, and the information indicating the next stipulation are transmitted to the first user terminal. The medical expenses, whether the current stipulation has been fulfilled or not, and the information indicating the next stipulation are output to the first user terminal. This allows the instant examinee to recognize the medical expenses, whether the current stipulation has been fulfilled or not, and the information indicating the next stipulation, and thus to recognize the relationship between the medical expenses and the fulfillment of the stipulation.

As a result, the instant examinee is given an incentive for self-management, to fulfill the next stipulation so as not to increase the medical expenses next time.

Further, the payment by the medical facility when providing the remote diagnosis is reduced as compared to the case of face-to-face diagnosis, as explained above, so the medical facility can set the medical expenses for the remote diagnosis lower than the medical expenses for the face-to-face diagnosis. As the medical expenses for the remote diagnosis is set lower than the medical expenses for the face-to-face diagnosis, the payment by the instant examinee is further reduced. Therefore, even in the case where continuous consultations are required as with a chronic disease, the medical expenses to be paid by the instant examinee are maintained relatively low, making it easier for the instant examinee to continue the consultations. The consultations thus continued would likely exert more treatment effects on the instant examinee.

As described above, according to the remote diagnostic aid system with the above configuration, the medical expenses are determined appropriately to reduce the payment by the examinee, while improving the treatment effects as appropriate.

In the remote diagnostic aid system of the present invention, it is preferable that
the stipulation is that the instant examinee is using a predetermined wearable device which automatically acquires a health condition of the instant examinee for transmission to the remote diagnostic aid system.

According to the remote diagnostic aid system with the above configuration, the medical expenses of the instant examinee are determined such that the medical expenses when using the wearable device become not higher than the medical expenses when not using the wearable device. This gives the instant examinee an incentive to use the wearable device.

Further, as the wearable device is used, the health conditions of the instant examinee may be automatically acquired and transmitted to the remote diagnostic aid system, leading to reduction in labor costs concerning acquisition of the health conditions of the instant examinee. This may reduce the payment by the medical institution as well.

In the remote diagnostic aid system of the present invention, it is preferable that
the stipulation is that the health condition of the instant examinee is a predetermined health condition.

According to the remote diagnostic aid system with the above configuration, the medical expenses are determined such that when the health condition of the instant examinee corresponds to the predetermined health condition, the medical expenses will become not higher than in the other case. This can give the instant examinee an incentive for managing the health so that the examinee's health condition will become the predetermined health condition.

In the remote diagnostic aid system with the above configuration, it is preferable that
the predetermined health condition is a health condition designated in advance by one or both of the instant examinee and the attending doctor.

According to the remote diagnostic aid system with the above configuration, the target health condition is set by one or both of the instant examinee and the attending doctor. This can offer a sense of responsibility to the instant examinee and, hence, to give the examinee a motivation for managing the health.

In the remote diagnostic aid system of the present invention, it is preferable that
the stipulation is that the health condition of the instant examinee continues to be a predetermined health condition for a certain period of time.

According to the remote diagnostic aid system with the above configuration, the medical expenses are reduced when the instant examinee's health condition continues to be a predetermined health condition for a certain period of time. This can give the instant examinee an incentive for managing the health continuously.

In the remote diagnostic aid system of the present invention, it is preferable that

the payment determination unit determines a payment of an organization such that the payment when the number of examinees associated with the organization is a predetermined number or greater becomes not higher than the payment when the number of examinees associated with the organization is smaller than the predetermined number.

According to the remote diagnostic aid system with the above configuration, the organization's payment becomes less as the number of examinees increases. This makes it easier for the organization to encourage the persons associated with the organization to have consultations. As a result, the number of examinees is likely to increase, so the sales in the entire system are assured even if the organization's payment of medical expenses per examinee is made small.

A remote diagnostic aid system according to the present invention is a network system including a remote diagnostic aid server and one or more doctor terminals communicating with a plurality of user terminals, and the remote diagnostic aid system includes:

a storage unit which stores available consultation time information including an available consultation time during which a doctor as a user of a respective one of the one or more doctor terminals is available for consultation;

a consultation appointment acceptance unit which receives, from a first user terminal as one of the plurality of user terminals, information including a desired consultation time during which an instant examinee as a user of the first user terminal wants to have a consultation, refers to the available consultation time information to extract an attending doctor who is available for consultation during the desired consultation time, and transmits, to one or both of the first user terminal and an attending doctor terminal as the doctor terminal of the attending doctor, communication establishment information for establishing a communication between the attending doctor terminal and the first user terminal;

a remote diagnostic aid unit which outputs examinee's statement information, received from the first user terminal with which the communication has been established on the basis of the communication establishment information, to the attending doctor terminal, and transmits doctor's statement information, input from the attending doctor terminal, to the first user terminal; and

a medical expense determination unit which recognizes a target action of the instant examinee on the basis of medical record information of the instant examinee received from the attending doctor terminal or the first user terminal, transmits the target action to the first user terminal, recognizes an adherence level of the instant examinee to the target action included in the medical record information, and determines medical expenses for the instant examinee such that the medical expenses when the adherence level is not lower than a predetermined adherence level become not higher than the medical expenses when the adherence level is lower than the predetermined adherence level.

According to the remote diagnostic aid system with the above configuration, the consultation appointment acceptance unit receives the information including the desired consultation time from the first user terminal and refers to the available consultation time information to extract an attending doctor who is available for consultation during the desired consultation time.

An appointment for consultation is thus made within the time desired by the instant examinee and during which the attending doctor is available. Even if the instant examinee cannot make time for receiving face-to-face diagnosis, the examinee can be given a diagnosis by designating the time for consultation. This alleviates the time-wise cost of the instant examinee. In addition, the consultation can be held at a time convenient for the attending doctor. Work opportunities are thus offered to doctors who have difficulties in performing medical examinations full time, like those having children.

The communication establishment information for establishing a communication between the attending doctor terminal and the first user terminal is transmitted by the consultation appointment acceptance unit to one or both of the attending doctor terminal and the first user terminal.

The examinee's statement information, received from the first user terminal with which the communication has been established on the basis of the communication establishment information, is output by the remote diagnostic aid unit to allow the attending doctor to recognize the examinee's statement information.

Further, the input doctor's statement information is transmitted by the remote diagnostic aid unit to the first user terminal. As this doctor's statement information is output at the first user terminal, the instant examinee can recognize the doctor's statement information input by the attending doctor.

In this manner, the remote diagnosis between the instant examinee and the attending doctor is aided.

Further, according to the remote diagnostic aid system with the above configuration, the target action included in the medical record information received from the doctor terminal is recognized, and the target action is transmitted to the first user terminal. Displaying this target action on the first user terminal allows the instant examinee to recognize the target action.

The medical expense determination unit analyzes the data input by the doctor, the data input by the instant examinee, or the medical record information, for example, to recognize the adherence level of the instant examinee to the target action included in the medical record information. The medical expense determination unit then determines the medical expenses for the instant examinee such that the medical expenses when the adherence level is not lower than a predetermined adherence level become not higher than the medical expenses when the adherence level is lower than the predetermined adherence level. This gives the instant examinee an incentive to adhere to the target action.

As described above, according to the remote diagnostic aid system with the above configuration, it is possible to give the instant examinee an incentive to adhere to the target action, to thereby encourage improvement in lifestyle.

In the remote diagnostic aid system of the present invention, it is preferable that the target action includes at least one of a target action related to eating or drinking, a target action related to exercise, and a target action related to medical equipment.

According to the remote diagnostic aid system with the above configuration, the target action includes those the instant examinee is expected to get interested in, such as those related to eating and drinking, exercise, and medical equipment, which can attract the instant examinee's interest. It is thus possible to encourage the instant examinee to adhere to the target action.

Brief Description of Drawings

FIG. 1 is an overall configuration diagram of a remote diagnostic aid system.

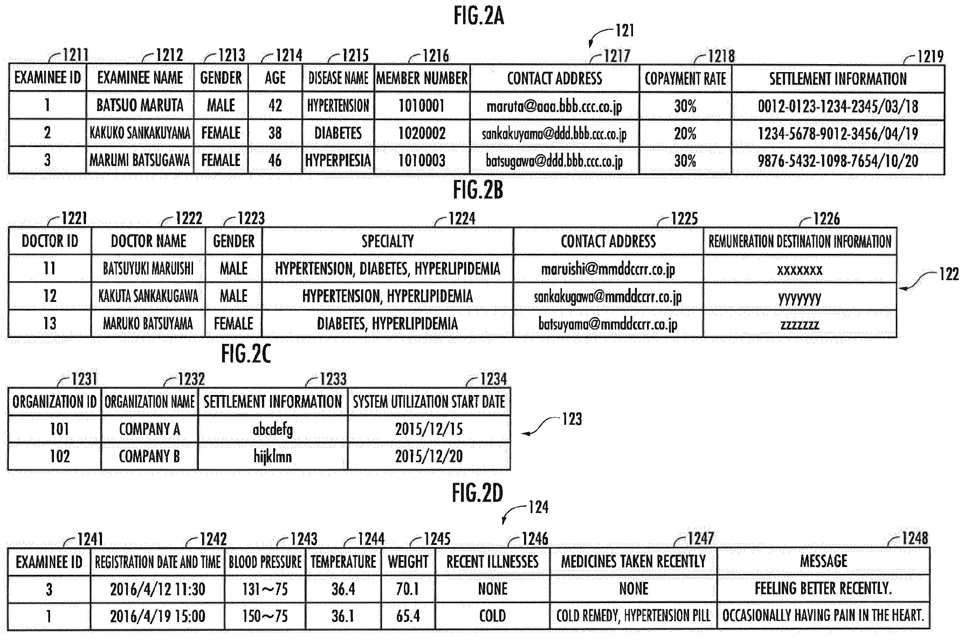

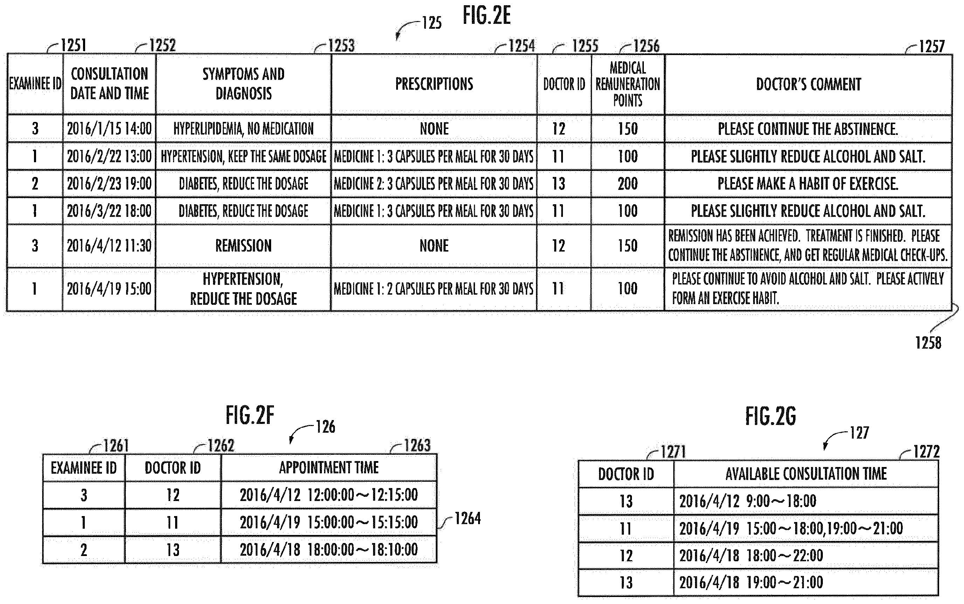

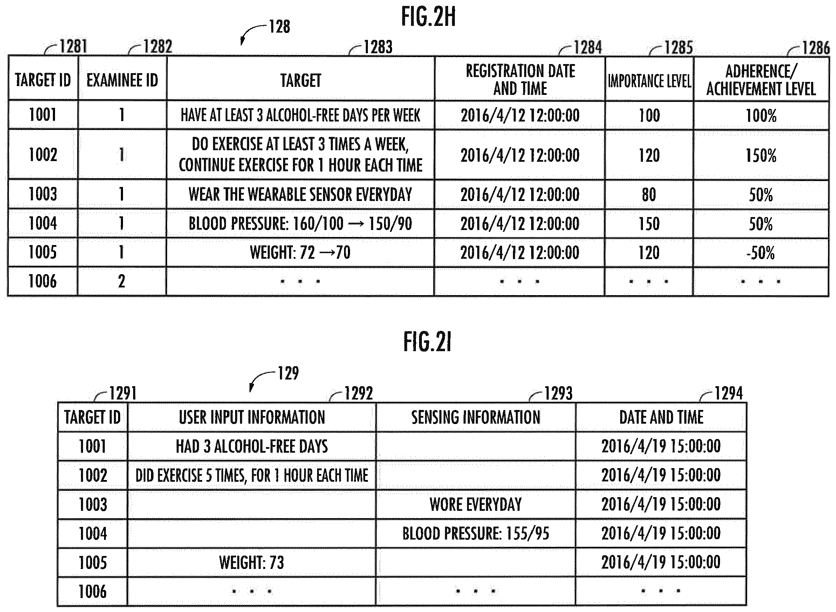

FIG. 2A shows an examinee list stored in a server storage unit, FIG. 2B shows a doctor list stored in the server storage unit, FIG. 2C shows an organization list stored in the server storage unit, FIG. 2D shows a diagnostic aid information list stored in the server storage unit, FIG. 2E shows an examination information list stored in the server storage unit, FIG. 2F shows an appointment information list stored in the server storage unit, FIG. 2G shows an available consultation time information list stored in the server storage unit, FIG. 2H shows a targets list stored in the server storage unit, and FIG. 2I shows a condition log stored in the server storage unit.

FIG. 3 is an overall flowchart illustrating a series of processing for remote diagnostic aid.

FIG. 4 is a flowchart illustrating appointment accepting processing.

FIG. 5 is a flowchart illustrating diagnostic aid information and sensing information registration processing.

FIG. 6A is the first half of a flowchart illustrating remote diagnostic aid processing, and FIG. 6B is the latter half of the flowchart illustrating the remote diagnostic aid processing.

FIG. 7A is a flowchart illustrating medical expense billing and remuneration paying processing, and FIG. 7B is a flowchart illustrating medical expense determination processing.

FIG. 8 is a configuration diagram of an examination information screen.

FIG. 9 is a flowchart illustrating organization's payment determination processing.

FIG. 10 is a flowchart illustrating processing of providing data on organization's payment of medical expenses.

FIG. 11A is a graph created from the data on the organization's payment of medical expenses, FIG. 11B is a graph created from first health condition data, and FIG. 11C is a graph created from second health condition data.

FIG. 12 is a flowchart illustrating processing of providing first health condition data.

FIG. 13 is a flowchart illustrating processing of providing second health condition data.

FIG. 14 shows an insurance organization's standard payment information list.

FIG. 15 is a flowchart illustrating medical expense billing and remuneration paying processing.

FIG. 16 is a flowchart illustrating organization's payment determination processing.

Description of Embodiments

(First Embodiment)

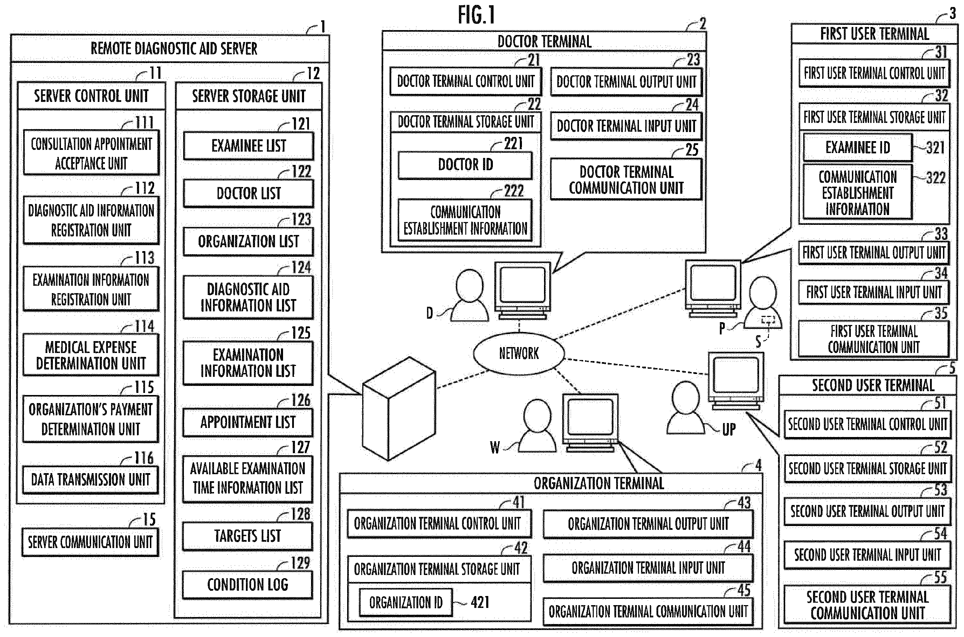

A remote diagnostic aid system according to a first embodiment of the present invention will be described with reference to FIGs. 1 to 13.

(Remote Diagnostic Aid System)

A remote diagnostic aid system is a system that aids remote diagnosis carried out between a doctor and an examinee.

As shown in FIG. 1, the remote diagnostic aid system includes a remote diagnostic aid server 1 and one or more doctor terminals 2. The remote diagnostic aid server 1 and the doctor terminal(s) 2 are each configured to be mutually communicable with respective ones of a first user terminal 3, a second user terminal 5, and one or more organization terminals 4 via a network such as the Internet. It should be noted that FIG. 1 shows one doctor terminal 2, one first user terminal 3, one organization terminal 4, and one second user terminal 5.

(Remote Diagnostic Aid Server)

The remote diagnostic aid server 1 includes: a server control unit 11, a server storage unit 12, and a server communication unit 15. It should be noted that a part or the whole of a computer constituting the remote diagnostic aid server 1 may be configured with a computer constituting another terminal such as a doctor terminal 2. For example, a part or the whole of the remote diagnostic aid server 1 may be configured with one or more doctor terminals 2 as mobile stations.

The server control unit 11 is configured with an arithmetic processing unit such as a central processing unit (CPU), a memory, and an input/output (I/O) device. The server control unit 11 may be configured with a single processor, or with a plurality of mutually communicable processors.

The server control unit 11 reads and executes a certain program to function as: a consultation appointment acceptance unit 111, a diagnostic aid information registration unit 112, an examination information registration unit 113, a medical expense determination unit 114, an organization's payment determination unit 115, and a data transmission unit 116, which perform arithmetic processing (described later).

The server storage unit 12 is configured with storage devices such as a read only memory (ROM), a random access memory (RAM), and a hard disk drive (HDD). The server storage unit 12 is configured to store computation results of the server control unit 11, or data received by the server control unit 11 via the server communication unit 15.

The server storage unit 12 is configured to store: an examinee list 121, a doctor list 122, an organization list 123, a diagnostic aid information list 124, an examination information list 125, an appointment list 126, an available consultation time information list 127, a targets list 128, and a condition log 129. It should be noted that information included in the examinee list 121, the diagnostic aid information list 124, the examination information list 125, the targets list 128, and the condition log 129 correspond to the "medical record information" in the present invention.

As shown in FIG. 2A, the examinee list 121 includes an examinee ID column 1211, an examinee name column 1212, a gender column 1213, an age column 1214, a disease name column 1215, a member number column 1216, a contact address column 1217, a copayment rate column 1218, and a settlement information column 1219.

The examinee ID column 1211 stores identifications (IDs) for identifying respective examinees. The examinee name column 1212 stores names of the respective examinees. The gender column 1213 stores genders of the respective examinees. The age column 1214 stores ages of the respective examinees. The disease name column 1215 stores disease names of the respective examinees. The member number column 1216 stores member numbers assigned to the respective examinees. Each member number includes an ID for identifying the organization to which the corresponding examinee belongs. The contact address column 1217 stores contact addresses, for example e-mail addresses, of the respective examinees. The copayment rate column 1218 stores respective examinees' copayment rates of medical expenses. The settlement information column 1219 stores information used for billing and settlement, such as credit card numbers and expiration dates, for the respective examinees.

The information included in the examinee list 121 may be input, for example, when an examinee registers with the remote diagnostic aid system.

The doctor list 122, as shown in FIG. 2B, includes a doctor ID column 1221, a doctor name column 1222, a gender column 1223, a specialty column 1224, a contact address column 1225, and a remuneration destination information column 1226.

The doctor ID column 1221 stores doctor IDs for identifying respective doctors. The doctor name column 1222 stores names of the respective doctors. The gender column 1223 stores genders of the respective doctors. The specialty column 1224 stores disease names included in the specialty areas of the respective doctors. Alternatively, the specialty column 1224 may store the names of the specialty areas (for example, "internal medicine") of the respective doctors. The contact address column 1225 stores contact addresses, for example e-mail addresses, of the respective doctors. The remuneration destination information column 1226 stores account information, for example, as information on destination of remuneration for the respective doctors. It should be noted that doctors are not limited to medical license holders; they may include dentists, nurse practitioners, and others with medical expertise.

The information included in the doctor list 122 is input, for example, when a doctor registers with the remote diagnostic aid system.

The organization list 123, as shown in FIG. 2C, includes an organization ID column 1231, an organization name column 1232, a settlement information column 1233, and a system utilization start date column 1234.

The organization ID column 1231 stores IDs for identifying respective organizations. The organization name column 1232 stores the names of the respective organizations. The settlement information column 1233 stores settlement information necessary for billing and settlement, such as withdrawal account information or credit card numbers, for the respective organizations. The system utilization start date column 1234 stores the dates when the respective organizations started utilizing the remote diagnostic aid system (for example, the date when a member belonging to the organization received remote diagnosis via the remote diagnostic aid system for the first time). An organization may be a company to which a user of the user terminal belongs, or it may be an insurance company tied up with the company to offer a private insurance system. The insurance company may offer, for example, an insurance plan in the case of utilizing a remote diagnostic system. This insurance plan may be a plan in which insurance premiums are further subdivided according to the age, symptoms, or other specific conditions of the users of the user terminals.

The diagnostic aid information list 124 is a list of diagnostic aid information registered by examinees before consultations. As shown in FIG. 2D, the list includes an examinee ID column 1241, a registration date and time column 1242, a blood pressure column 1243, a temperature column 1244, a weight column 1245, a recent illnesses column 1246, a medicines taken recently column 1247, and a message column 1248. The diagnostic aid information list 124 may include other columns including information necessary for consultations.

The examinee ID column 1241 stores an examinee ID. The registration date and time column 1242 stores the date and time when information has been registered. The blood pressure column 1243 stores a blood pressure value. The temperature column 1244 stores a value indicating the body temperature. The weight column 1245 stores a value indicating the body weight. The recent illnesses column 1246 stores character strings indicating disease names. The medicines taken recently column 1247 stores character strings indicating medicines. The message column 1248 stores character strings input by an examinee.

The examination information list 125 stores a list of examination information regarding results of examinations in remote diagnosis. As shown in FIG. 2E, the list includes an examinee ID column 1251, a consultation date and time column 1252, a symptoms and diagnosis column 1253, a prescriptions column 1254, a doctor ID column 1255, a medical remuneration point column 1256, and a doctor's comment column 1257.

The examinee ID column 1251 stores an examinee ID of the examinee (instant examinee) to be examined. The consultation date and time column 1252 stores the date and time when the instant examinee received a remote diagnosis. The symptoms and diagnosis column 1253 stores doctor's opinion information on the instant examinee at the remote diagnosis. The prescriptions column 1254 stores the name and dosage of medicines prescribed for the instant examinee. The doctor ID column 1255 stores a doctor ID of the doctor (attending doctor) who examined the instant examinee. The medical remuneration point column 1256 stores medical remuneration points for the instant examinee at the remote diagnosis. The doctor's comment column 1257 stores the attending doctor's comment to the instant examinee.

The appointment list 126 includes, as shown in FIG. 2F, an examinee ID column 1261, a doctor ID column 1262, and an appointment time column 1263.

The examinee ID column 1261 stores an ID of the examinee who has made an appointment for a remote diagnosis. The doctor ID column 1262 stores an ID of the doctor who is in charge of the remote diagnosis. The appointment time column 1263 stores a consultation appointment time for the remote diagnosis.

The available consultation time information list 127 is a list of available consultation time information regarding times during which the respective doctors are available for consultation. As shown in FIG. 2G, the list includes a doctor ID column 1271 and an available consultation time column 1272.

The doctor ID column 1271 stores doctor IDs of respective doctors. The available consultation time column 1272 stores available consultation times when the respective doctors are available for consultation.

The targets list 128, as shown in FIG. 2H, includes a target ID column 1281, an examinee ID column 1282, a target column 1283, a registration date and time column 1284, an importance level column 1285, and an adherence/achievement level column 1286.

The target ID column 1281 stores target IDs of respective targets. The examinee ID column 1282 stores examinee IDs of respective examinees. The target column 1283 stores targets for the respective examinees. The registration date and time column 1284 stores the date and time when a target has been registered. The importance level column 1285 stores the level of importance of the target. The adherence/achievement level column 1286 stores the level of adherence to or the level of achievement of the target.

The target includes, for example, a target action of an examinee such as "abstinence from alcohol", "exercise", or "sleep at 22:00", for example. The target may also be expressed as a combination with one or both of: the target frequency or target number of times of doing the target action, such as "at least three times a week" or "everyday"; and the degree of the target action, such as "do exercise continuously for one hour". Further, in place of or in addition to the examinee's state of activity expressed by target action, target frequency or target number of times, or degree of target action, the target may be one regarding the examinee's state of wearing wearable equipment, or one regarding the examinee's health conditions expressed by a target value of blood pressure or other biological information of the examinee.

The importance level of a target is expressed by a positive integer, for example.

The level of adherence to or level of achievement of a target is expressed by a numerical value. For example, it may be set such that the adherence/achievement level of 100 means that the target has been achieved 100%. In this case, if the final state is farther from the target than at the beginning, the adherence/achievement level may be set to zero or to a negative value. If the final state has exceeded the target, the adherence/achievement level may be set to 100 or to a numerical value exceeding 100.

The condition log 129, as shown in FIG. 2I, includes a target ID column 1291, a user input information column 1292, a sensing information column 1293, and a date and time column 1294.

The target ID column 1291 stores target IDs of respective targets. The user input information column 1292 includes information input from an examinee, such as a combination of target action, frequency or number of times of execution of the target action, and degree of execution of the target action, or a value of the examiner's weight or the like. The sensing information column 1293 includes sensing information received from the wearable sensor S, such as the active state of the wearable sensor S, the frequency or number of times of wearing the sensor estimated from the sensing information, or the sensing information itself. The date and time column 1294 includes the date and time when the user input information was input or the date and time when the sensing information was received.

The server communication unit 15 is configured with a communication device which is connected to a public communication network (for example, the Internet) as a network to communicate with external terminals such as a doctor terminal 2, a first user terminal 3, an organization terminal 4, and a second user terminal 5.

(Doctor Terminal)

A doctor terminal 2 includes: a doctor terminal control unit 21, a doctor terminal storage unit 22, a doctor terminal output unit 23, a doctor terminal input unit 24, and a doctor terminal communication unit 25.

The doctor terminal 2 is configured with a computer, such as a desktop computer, which has its size, shape, and weight designed to be positioned in a specific place. The doctor terminal 2 may be a computer, such as a tablet terminal or a smartphone, which has its size, shape, and weight designed so that it can be carried along by a doctor D as the user of the doctor terminal 2.

The doctor terminal control unit 21 is configured with an arithmetic processing unit such as a CPU, a memory, and an I/O device. The doctor terminal control unit 21 has installed therein a remote diagnostic aid program downloaded from the remote diagnostic aid server 1. The doctor terminal control unit 21 is configured to perform arithmetic processing (described later) as the remote diagnostic aid program is started.

The doctor terminal storage unit 22 is configured with, for example, a storage device such as a ROM, a RAM, a HDD, etc. The doctor terminal storage unit 22 is configured to store a doctor ID 221 of a doctor D registered in advance, and communication establishment information 222 which is stored in appointment accepting processing (described later).

The doctor terminal output unit 23 is configured with a display device such as a liquid crystal panel, and the doctor terminal input unit 24 is configured with a position input device such as a touch pad. These in combination constitute a touch panel. Additionally or alternatively, the doctor terminal input unit 24 may include a keyboard. The doctor terminal output unit 23 further includes a speaker. The doctor terminal input unit 24 further includes a microphone.

The doctor terminal communication unit 25 is configured to mutually communicate with external terminals such as the remote diagnostic aid server 1 via wired communication or wireless communication according to the communication standard of WiFi (registered trademark) or the like.

(First User Terminal)

A first user terminal 3 includes: a first user terminal control unit 31, a first user terminal storage unit 32, a first user terminal output unit 33, a first user terminal input unit 34, and a first user terminal communication unit 35.

The first user terminal control unit 31 has installed therein, instead of the remote diagnostic aid program, a remote diagnostic consultation aid program downloaded from an organization terminal 4 (or a server of the organization to which the instant examinee P belongs, or the remote diagnostic aid server 1). The first user terminal control unit 31 is configured to perform arithmetic processing (described later) as the remote diagnostic consultation aid program is started.

The first user terminal storage unit 32 stores, instead of the doctor ID 221 and the communication establishment information 222, an examinee ID 321 of the instant examinee P as a user of the first user terminal 3, and communication establishment information 322. Otherwise, the first user terminal 3 has a configuration similar to that of the doctor terminal 2.

Further, the wearable sensor S is configured to be wearable by the instant examinee P. The wearable sensor S is configured to recognize biological information or behavioral information of the instant examinee P and the wearing state of the wearable sensor S, and transmit the biological information to the remote diagnostic aid server 1 at regular intervals or in response to a manipulation by the instant examinee P.

The biological information of the instant examinee P may include, for example, blood pressure, heart rate, breathing rate, temperature, weight, and height of the instant examinee P.

The behavioral information of the instant examinee P may include, for example, the number of steps of the instant examinee P, the length of time the instant examinee P walked, and the length of time the instant examinee P ran.

The wearable sensor S may have an independent communication unit and transmit the biological information to the remote diagnostic aid server 1 via the communication unit. Alternatively, the wearable sensor S may be connected to the first user terminal 3 via a USB, for example, and transmit the biological information to the remote diagnostic aid server 1 via the first user terminal communication unit 35.

(Organization Terminal)

An organization terminal 4 includes: an organization terminal control unit 41, an organization terminal storage unit 42, an organization terminal output unit 43, an organization terminal input unit 44, and an organization terminal communication unit 45.

The organization terminal 4 has a configuration similar to that of the doctor terminal 2 except that no remote diagnostic aid program has been installed, that the organization terminal storage unit 42 stores an organization ID 421 of the organization instead of the doctor ID 221, and that the organization terminal storage unit 42 does not store the communication establishment information 222.

(Second User Terminal)

A second user terminal 5 includes: a second user terminal control unit 51, a second user terminal storage unit 52, a second user terminal output unit 53, a second user terminal input unit 54, and a second user terminal communication unit 55.

The second user terminal 5 has a configuration similar to that of the doctor terminal 2 except that no remote diagnostic aid program has been installed and that the second user terminal storage unit 52 does not store the doctor ID 221 or the communication establishment information 222.

(General Outline of Series of Processing for Remote Diagnostic Aid)

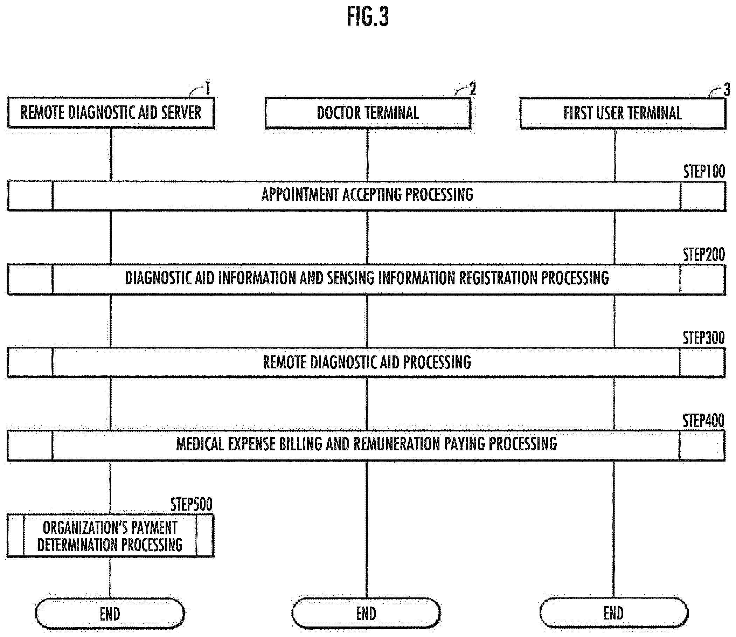

A general outline of a series of processing for remote diagnostic aid will be given with reference to FIG. 3.

The remote diagnostic aid server 1, the doctor terminal 2, and the first user terminal 3 perform appointment accepting processing described later (STEP 100/FIG. 3). Through the appointment accepting processing, an appointment is made for the consultation date and time for a remote diagnosis, and communication establishment information is transmitted to the doctor terminal 2 and the first user terminal 3 for establishing a communication therebetween.

The remote diagnostic aid server 1, the doctor terminal 2, and the first user terminal 3 perform diagnostic aid information registration processing and sensing information registration processing described later (STEP 200/FIG. 3). With the diagnostic aid information and sensing information registration processing, diagnostic aid information for an instant examinee P is additionally registered in the diagnostic aid information list 124 and sensing information is additionally registered in the condition log 129 before the examinee receives remote diagnosis, so that the information is shared with an attending doctor D who is in charge of remote diagnosis of the instant examinee P.

The remote diagnostic aid server 1, the doctor terminal 2, and the first user terminal 3 perform remote diagnostic aid processing described later (STEP 300/FIG. 3). The doctor terminal 2 and the first user terminal 3 establish a communication using the communication establishment information to aid the remote diagnosis between an attending doctor D and an instant examinee P. When the remote diagnosis is finished, the doctor terminal 2 transmits examination information for the instant examinee P to the remote diagnostic aid server 1, so as to be added to the examination information list 125. Further, through the communication between the remote diagnostic aid server 1 and the first user terminal 3, a next target of the instant examinee P is added to the targets list 128.

The remote diagnostic aid server 1, the doctor terminal 2, and the first user terminal 3 perform medical expense billing and remuneration paying processing described later (STEP 400/FIG. 3). The remote diagnostic aid server 1 recognizes medical expenses for the remote diagnosis on the basis of the examination information and the target information registered in STEP 300/FIG. 3, and charges a payment facility for a practice cost of the remote diagnosis, charges the instant examinee P for a copayment of the remote diagnosis, and settles the payment of remuneration to the attending doctor D.

The remote diagnostic aid server 1 performs organization's payment determination processing described later (STEP 500/FIG. 3). The remote diagnostic aid server 1 uses settlement information of the organization to make settlement of the organization's payment.

(Appointment Accepting Processing)

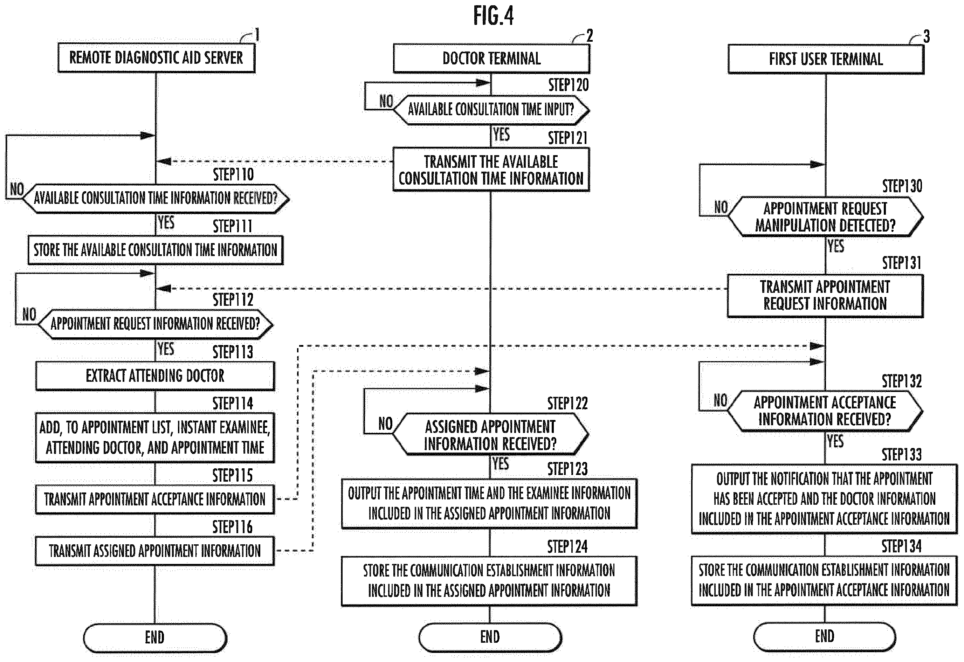

The appointment accepting processing will be described with reference to FIG. 4.

Firstly, the doctor terminal control unit 21 determines whether an available consultation time has been input from the attending doctor D via the doctor terminal input unit 24 (STEP 120/FIG. 4).

If the determination result is negative ("NO" in STEP 120/FIG. 4), the doctor terminal control unit 21 performs the process in STEP 120/FIG. 4 again.

If the determination result is positive ("YES" in STEP 120/FIG. 4), the doctor terminal control unit 21 creates available consultation time information including the doctor ID of the attending doctor D, registered in advance in the doctor terminal storage unit 22, and the available consultation time, input from the attending doctor D, and transmits the available consultation time information to the remote diagnostic aid server 1 via the doctor terminal communication unit 25 (STEP 121/FIG. 4).

The consultation appointment acceptance unit 111 determines whether available consultation time information has been received via the server communication unit 15 (STEP 110/FIG. 4).

If the determination result is negative ("NO" in STEP 110/FIG. 4), the consultation appointment acceptance unit 111 performs the process in STEP 110/FIG. 4 again.

If the determination result is positive ("YES" in STEP 110/FIG. 4), the consultation appointment acceptance unit 111 stores the received available consultation time information in the available consultation time information list 127 shown in FIG. 2G (STEP 111/FIG. 4).

The first user terminal control unit 31 determines whether an appointment request manipulation from an instant examinee P designating a desired consultation time has been detected via the first user terminal input unit 34 (STEP 130/FIG. 4).

It should be noted that prior to the STEP 130/FIG. 4, the consultation appointment acceptance unit 111 accessed by the first user terminal 3 may refer to the examinee list 121 to recognize the name of the disease of the instant examinee P, refer to the doctor list 122 to extract doctors who specialize in the disease, refer to the available consultation time information list 127 to extract the available consultation times of the extracted doctors, and transmit the available consultation times to the first user terminal 3 for output to the first user terminal output unit 33.

If the determination result is negative ("NO" in STEP 130/FIG. 4), the first user terminal control unit 31 performs the process in STEP 130/FIG. 4 again.

If the determination result is positive ("YES" in STEP 130/FIG. 4), the first user terminal control unit 31 creates appointment request information including the examinee ID of the instant examinee P, registered in advance in the first user terminal storage unit 32, and a desired consultation time, input from the instant examinee P, and transmits the appointment request information to the remote diagnostic aid server 1 via the first user terminal communication unit 35 (STEP 131/FIG. 4).

The consultation appointment acceptance unit 111 determines whether appointment request information has been received via the server communication unit 15 (STEP 112/FIG. 4).

If the determination result is negative ("NO" in STEP 112/FIG. 4), the consultation appointment acceptance unit 111 performs the process in STEP 112/FIG. 4 again.

If the determination result is positive ("YES" in STEP 112/FIG. 4), the consultation appointment acceptance unit 111, on the basis of the appointment request information, refers to the examinee list 121, the doctor list 122, and the available consultation time information list 127 to extract an attending doctor who is available in the desired consultation time indicated in the appointment request information (STEP 113/FIG. 4).

More specifically, the consultation appointment acceptance unit 111 refers to the examinee list 121 to recognize, using the examinee ID of the instant examinee P included in the appointment request information, the name of the disease of the instant examinee P. The consultation appointment acceptance unit 111 then refers to the doctor list 122 and the available consultation time information list 127 to recognize an attending doctor who can handle the disease of the instant examinee P and who is available in the desired consultation time included in the appointment request information.

For example, in the case where the instant examinee P is "Batsuo MARUTA" and the desired consultation time is "2016/4/19 15:00:00 to 15:15:00", the consultation appointment acceptance unit 111 refers to the examinee list 121 to recognize the disease name "hypertension" of the instant examinee P by using the examinee ID "1" of the instant examinee P "Batsuo MARUTA" included in the appointment request information. The consultation appointment acceptance unit 111 then refers to the doctor list 122 and the available consultation time information list 127 to recognize, as an attending doctor, a doctor "Batsuyuki MARUISHI" who specializes in "hypertension" as the disease name of the instant examinee P and who is available in the desired consultation time of "2016/4/19 15:00:00 to 15:15:00". It should be noted that the consultation appointment acceptance unit 111 may recognize, as an attending doctor, a doctor who is available based on the available consultation time information list 127 and the desired consultation time.

The consultation appointment acceptance unit 111 adds, to the appointment list 126, the examinee ID of the instant examinee P, the doctor ID of the attending doctor, and the appointment time as the desired consultation time (STEP 114/FIG. 4).

For example, as shown in the field 1264 in FIG. 2F, the consultation appointment acceptance unit 111 adds, to the appointment list 126, the examinee ID "1" of the instant examinee P "Batsuo MARUTA", the doctor ID "11" of the attending doctor "Batsuyuki MARUISHI", and the appointment time "2016/4/19 15:00:00 to 15:15:00" as the desired consultation time.

The consultation appointment acceptance unit 111 refers to the examinee list 121 to recognize the contact address of the instant examinee P, and transmits to the contact address of the instant examinee P, via the server communication unit 15, appointment acceptance information which includes a notification that the appointment has been accepted, communication establishment information with respect to the attending doctor, and doctor information of the attending doctor (STEP 115/FIG. 4).

Here, the communication establishment information with respect to the attending doctor is, for example, information necessary for receiving remote diagnosis by the attending doctor, which information includes, for example, the doctor ID of the attending doctor, and information on the contact address of the attending doctor as information for using a video phone service by the remote diagnostic aid server 1 or information for using an external video phone service.

The first user terminal control unit 31 determines whether appointment acceptance information has been received via the first user terminal communication unit 35 (STEP 132/FIG. 4).

If the determination result is negative ("NO" in STEP 132/FIG. 4), the first user terminal control unit 31 performs the process in STEP 132/FIG. 4 again.

If the determination result is positive ("YES" in STEP 132/FIG. 4), the first user terminal control unit 31 outputs the notification that the appointment has been accepted and the doctor information of the attending doctor, included in the appointment acceptance information, to the first user terminal output unit 33 (STEP 133/FIG. 4), and stores the communication establishment information, included in the appointment acceptance information, in the first user terminal storage unit 32 (STEP 134/FIG. 4).

The consultation appointment acceptance unit 111 refers to the doctor list 122 to recognize the contact address of the attending doctor D, and transmits to the contact address of the attending doctor D, via the server communication unit 15, assigned appointment information which includes an appointment time, communication establishment information with respect to an instant examinee P, and examinee information of the instant examinee P (STEP 116/FIG. 4).

Here, the communication establishment information with respect to the instant examinee P is, for example, information necessary for receiving remote diagnosis by the attending doctor D, which is similar to the communication establishment information with respect to the attending doctor.

The doctor terminal control unit 21 determines whether assigned appointment information has been received via the doctor terminal communication unit 25 (STEP 122/FIG. 4).

If the determination result is negative ("NO" in STEP 122/FIG. 4), the doctor terminal control unit 21 performs the process in STEP 122/FIG. 4 again.

If the determination result is positive ("YES" in STEP 122/FIG. 4), the doctor terminal control unit 21 outputs the appointment time and the examinee information of the instant examinee P, included in the assigned appointment information, to the doctor terminal output unit 23 (STEP 123/FIG. 4), and stores the communication establishment information, included in the assigned appointment information, in the doctor terminal storage unit 22 (STEP 124/FIG. 4).

This completes the appointment accepting processing. It should be noted that the processes in STEP 115 and STEPs 132 to 134 in FIG. 4 may be omitted, or the processes in STEP 116 and STEPs 122 to 124 in FIG. 4 may be omitted.

(Diagnostic Aid Information and Sensing Information Registration Processing)

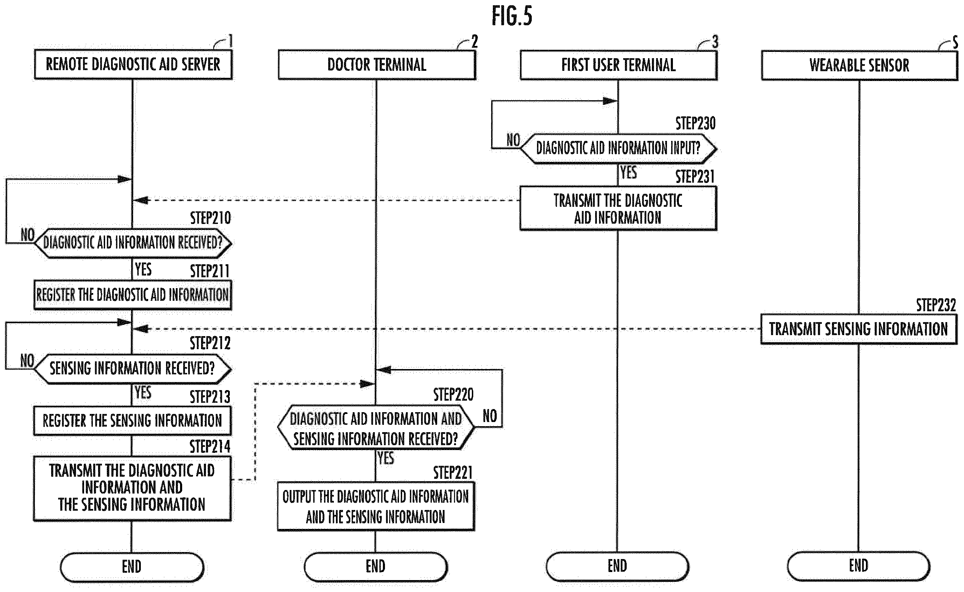

The diagnostic aid information and sensing information registration processing will be described with reference to FIG. 5.

The first user terminal control unit 31 determines whether diagnostic aid information has been input via the first user terminal input unit 34 (STEP 230/FIG. 5). The diagnostic aid information, as shown in FIG. 2D, is information including the examinee ID and information input by the instant examinee P, such as the weight and the like, recent illnesses, medicines taken recently, and message from the instant examinee P, which information can be referred to by a doctor upon consultation.

If the determination result is negative ("NO" in STEP 230/FIG. 5), the first user terminal control unit 31 performs the process in STEP 230/FIG. 5 again. The examinee ID may be input by the instant examinee P, or read from the first user terminal storage unit 32.

If the determination result is positive ("YES" in STEP 230/FIG. 5), the first user terminal control unit 31 transmits the diagnostic aid information to the remote diagnostic aid server 1 via the first user terminal communication unit 35 (STEP 231/FIG. 5).

The diagnostic aid information registration unit 112 determines whether diagnostic aid information has been received via the server communication unit 15 (STEP 210/FIG. 5).

If the determination result is negative ("NO" in STEP 210/FIG. 5), the diagnostic aid information registration unit 112 performs the process in STEP 210/FIG. 5 again.

If the determination result is positive ("YES" in STEP 210/FIG. 5), the diagnostic aid information registration unit 112 adds the diagnostic aid information to the diagnostic aid information list 124 and the condition log 129 for registration (STEP 211/FIG. 5).

The wearable sensor S transmits sensing information, including measured or estimated conditions of the instant examinee P, to the remote diagnostic aid server 1 (STEP 232/FIG. 5). The measured or estimated conditions of the instant examinee P may include, for example, biological information such as blood pressure, heart rate, breathing rate, or temperature, or the wearing state of the wearable sensor S estimated from the biological information or from the operating state of the sensor.

The diagnostic aid information registration unit 112 determines whether sensing information has been received via the server communication unit 15 (STEP 212/FIG. 5).

If the determination result is negative ("NO" in STEP 212/FIG. 5), the diagnostic aid information registration unit 112 performs the process in STEP 212/FIG. 5 again.

If the determination result is positive ("YES" in STEP 212/FIG. 5), the diagnostic aid information registration unit 112 adds the sensing information to the condition log 129 for registration (STEP 213/FIG. 5). The diagnostic aid information registration unit 112 refers to the appointment list 126 to recognize the attending doctor D, on the basis of the examinee ID included in the diagnostic aid information, and refers to the doctor list 122 to recognize the contact address of the attending doctor D, and then transmits the diagnostic aid information and the sensing information to the contact address of the attending doctor D (STEP 214/FIG. 5).

The doctor terminal control unit 21 determines whether diagnostic aid information and sensing information have been received (STEP 220/FIG. 5).

If the determination result is negative ("NO" in STEP 220/FIG. 5), the doctor terminal control unit 21 performs the process in STEP 220/FIG. 5 again.

If the determination result is positive ("YES" in STEP 220/FIG. 5), the doctor terminal control unit 21 outputs the diagnostic aid information and the sensing information to the doctor terminal output unit 23 (STEP 221/FIG. 5).

This completes the diagnostic aid information and sensing information registration processing.

(Remote Diagnostic Aid Processing)

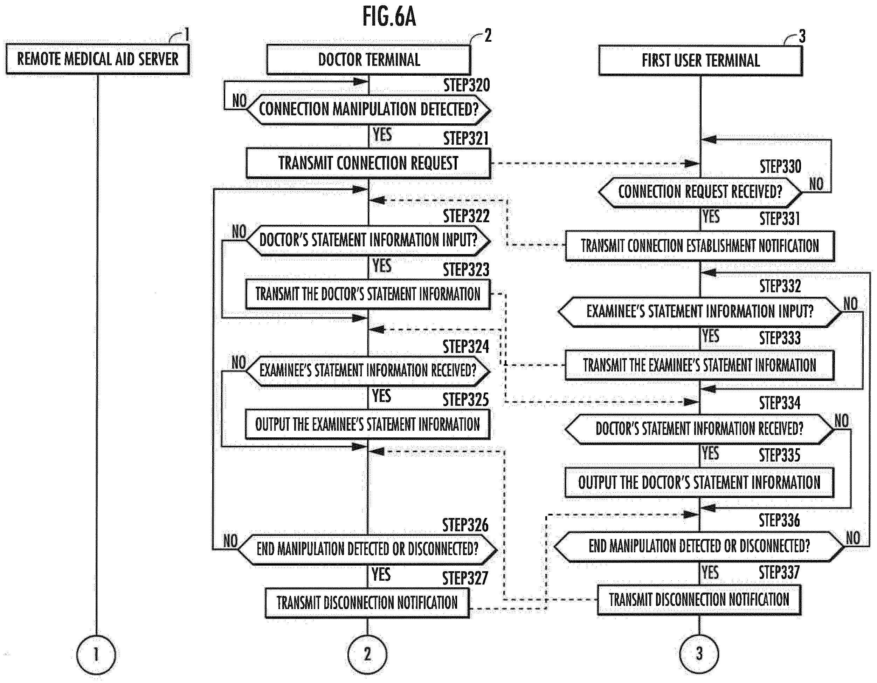

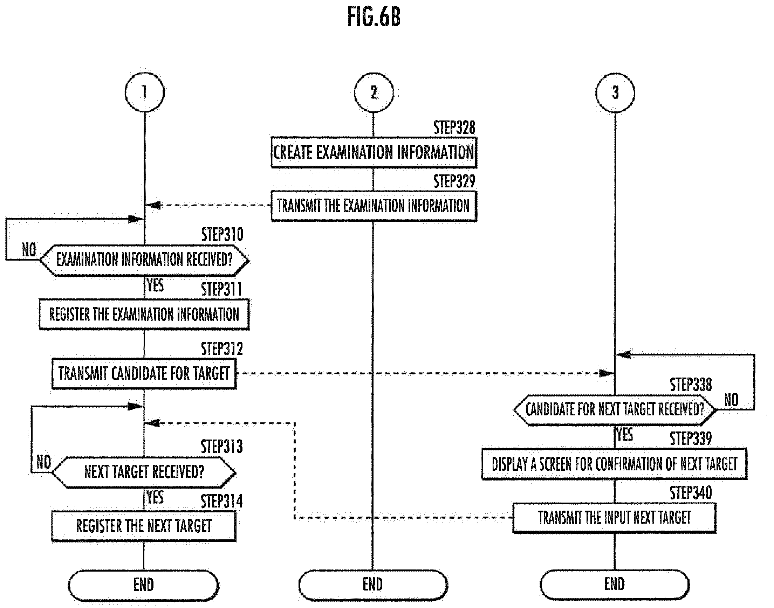

The remote diagnostic aid processing will be described with reference to FIGs. 6A and 6B.

The doctor terminal control unit 21 determines whether a connection manipulation from the attending doctor D has been detected via the doctor terminal input unit 24 (STEP 320/FIG. 6A).

If the determination result is negative ("NO" in SETP 320/FIG. 6A), the doctor terminal control unit 21 performs the process in SETP 320/FIG. 6A again.

If the determination result is positive ("YES" in SETP 320/FIG. 6A), the doctor terminal control unit 21 creates a connection request from the communication establishment information 222 stored in the doctor terminal storage unit 22, and transmits the connection request to the first user terminal 3 via the doctor terminal communication unit 25 (STEP 321/FIG. 6A).

The first user terminal control unit 31 determines whether a connection request has been received via the first user terminal communication unit 35 (STEP 330/FIG. 6A).

If the determination result is negative ("NO" in SETP 330/FIG. 6A), the first user terminal control unit 31 performs the process in SETP 330/FIG. 6A again.

If the determination result is positive ("YES" in SETP 330/FIG. 6A), the first user terminal control unit 31 stores the connection request in the first user terminal storage unit 32, and transmits a connection establishment notification notifying that the connection has been established, to the doctor terminal 2 (STEP 331/FIG. 6A).

The doctor terminal control unit 21 determines whether doctor's statement information has been input via the doctor terminal input unit 24 (STEP 322/FIG. 6A). For example, the doctor terminal control unit 21 may recognize utterance of the attending doctor D, input via the microphone as the doctor terminal input unit 24, as the doctor's statement information. Alternatively, for example, the doctor terminal control unit 21 may recognize character strings or the like, input via manipulations of the keyboard or touch panel as the doctor terminal input unit 24, as the doctor's statement information.

If the determination result is positive ("YES" in STEP 322/FIG. 6A), the doctor terminal control unit 21 transmits the doctor's statement information to the first user terminal 3 via the doctor terminal communication unit 25 (STEP 323/FIG. 6A).

If the determination result is negative ("NO" in STEP 322/FIG. 6A), the doctor terminal control unit 21 performs a process in STEP 324/FIG. 6A.

Following the STEP 331/FIG. 6A, the first user terminal control unit 31 determines whether examinee's statement information has been input via the first user terminal input unit 34 (STEP 332/FIG. 6A).

For example, the first user terminal control unit 31 may recognize utterance of the instant examinee P, input via the microphone as the first user terminal input unit 34, as the examinee's statement information. Alternatively, for example, the first user terminal control unit 31 may recognize character strings or the like, input via manipulations of the keyboard or touch panel as the first user terminal input unit 34, as the examinee's statement information.

If the determination result is positive ("YES" in STEP332/FIG. 6A), the first user terminal control unit 31 transmits the examinee's statement information to the doctor terminal 2 via the first user terminal communication unit 35 (STEP 333/FIG. 6A).

If the determination result is negative ("NO" in STEP 332/FIG. 6A), the first user terminal control unit 31 performs a process in STEP 334/FIG. 6A.

In STEP 324/FIG. 6A, the doctor terminal control unit 21 determines whether examinee's statement information has been received from the first user terminal 3 via the doctor terminal communication unit 25.

If the determination result is positive ("YES" in STEP 324/FIG. 6A), the doctor terminal control unit 21 outputs the received examinee's statement information to the doctor terminal output unit 23 (STEP 325/FIG. 6A).

If the determination result is negative ("NO" in STEP 324/FIG. 6A), or following the process in STEP 325/FIG. 6A, the doctor terminal control unit 21 performs a process in STEP 326/FIG. 6A.

In STEP 326/FIG. 6A, the doctor terminal control unit 21 determines whether an end manipulation has been detected via the doctor terminal input unit 24 or the connection with the first user terminal 3 has been disconnected.

If the determination result is positive ("YES" in STEP 326/FIG. 6A), the doctor terminal control unit 21 performs a process in STEP 327/FIG. 6A.

If the determination result is negative ("NO" in STEP 326/FIG. 6A), the doctor terminal control unit 21 performs the process in STEP 322/FIG. 6A.

In STEP 334/FIG. 6A, the first user terminal control unit 31 determines whether doctor's statement information has been received from the doctor terminal 2 via the first user terminal communication unit 35.

If the determination result is positive ("YES" in STEP 334/FIG. 6A), the first user terminal control unit 31 outputs the received doctor's statement information to the first user terminal output unit 33 (STEP 335/FIG. 6A).

If the determination result is negative ("NO" in STEP 334/FIG. 6A), or following the process in STEP 335/FIG. 6A, the first user terminal control unit 31 performs a process in STEP 336/FIG. 6A.

In STEP 336/FIG. 6A, the first user terminal control unit 31 determines whether an end manipulation has been detected via the first user terminal input unit 34 or the connection with the doctor terminal 2 has been disconnected.

If the determination result is positive ("YES" in STEP 336/FIG. 6A), the first user terminal control unit 31 transmits a disconnection notification to the doctor terminal 2 via the first user terminal communication unit 35 (STEP 337/FIG. 6A).

If the determination result is negative ("NO" in STEP 336/FIG. 6A), the first user terminal control unit 31 performs the process in STEP 332/FIG. 6A.

In STEP 327/FIG. 6A, the doctor terminal control unit 21 transmits a disconnection notification to the first user terminal 3 via the doctor terminal communication unit 25.

The doctor terminal control unit 21 creates examination information for the instant examinee P on the basis of information input via the doctor terminal input unit 24 (STEP 328/FIG. 6B). The information input via the doctor terminal input unit 24 includes symptoms and diagnosis, prescriptions, medical remuneration points, and doctor's comment. The examination information for the instant examinee P includes the examinee ID included in the communication establishment information, consultation date and time at which a connection establishment notification was received, symptoms and diagnosis, prescriptions, doctor ID of the attending doctor D, medical remuneration points, doctor's comment, a candidate for target of the instant examinee P, and the adherence/achievement level of a target of the instant examinee P. Among these, the medical remuneration points are points according to the contents of medical examination performed by the doctor. The medical remuneration points may be those used in a typical insurance system.

For example, the examination information for the instant examinee P "Batsuo MARUTA" includes: the examinee ID "1", the consultation date and time "2016/4/19 15:00:00", the symptoms and diagnosis "hypertension, reduce the dosage", the prescriptions "Medicine 1: 2 capsules per meal for 30 days", the doctor ID "11" of the attending doctor D, the medical remuneration points "100", the doctor's comment "Please continue to avoid alcohol and salt. Please actively form an exercise habit", a candidate for next target of the instant examinee P "Have at least 3 alcohol-free days per week" and its importance level "100", and the adherence/achievement level "100" for the current target of the instant examinee P "Have at least 3 alcohol-free days per week".

A candidate for next target of the instant examinee P includes a target in the same form as the one included in the target column 1283, and the importance level of the target. The importance level of a target may be designated by a doctor, or determined in advance. The adherence/achievement level of the current target of the instant examinee P may be input by the doctor, or may be determined on the basis of the user input information or the sensing information.

The doctor terminal control unit 21 transmits the created examination information to the remote diagnostic aid server 1 via the doctor terminal communication unit 25 (STEP 329/FIG. 6B).

It should be noted that the examination information input from the instant examinee P to the first user terminal 3 may be transmitted to the remote diagnostic aid server 1.

The examination information registration unit 113 determines whether examination information has been received via the server communication unit 15 (STEP 310/FIG. 6B).

If the determination result is negative ("NO" in STEP 310/FIG. 6B), the examination information registration unit 113 performs the process in STEP 310/FIG. 6B again.

If the determination result is positive ("YES" in STEP 310/FIG. 6B), the examination information registration unit 113 adds part of the received examination information other than the candidate for next target and the adherence/achievement level of the current target, to the examination information list 125, and registers the adherence/achievement level of the current target in the targets list 128 (STEP 311/FIG. 6B). For example, the field 1258 shown in FIG. 2E is added to the examination information list 125 as the examination information for the instant examinee P "Batsuo MARUTA". Further, the adherence/achievement level of the current target is registered in the adherence/achievement level column 1286 on the targets list 128, in the position corresponding to the target ID.

The examination information registration unit 113 transmits the candidate for next target to the first user terminal 3 via the server communication unit 15 (STEP 312/FIG. 6B).

The first user terminal control unit 31 determines whether a candidate for next target has been received (STEP 338/FIG. 6B).

If the determination result is negative ("NO" in STEP 338/FIG. 6B), the first user terminal control unit 31 performs the process in STEP 338/FIG. 6B again.

If the determination result is positive ("YES" in STEP 338/FIG. 6B), the first user terminal control unit 31 displays a screen for confirmation of next target (STEP 339/FIG. 6B).

The first user terminal control unit 31 transmits a next target, input via the first user terminal input unit 34, to the remote diagnostic aid server 1 via the first user terminal communication unit 35 (STEP 340/FIG. 6B).

The examination information registration unit 113 determines whether a next target has been received via the server communication unit 15 (STEP 313/FIG. 6B).

If the determination result is negative ("NO" in STEP 313/FIG. 6B), the examination information registration unit 113 performs the process in STEP 313/FIG. 6B again.

If the determination result is positive ("YES" in STEP 313/FIG. 6B), the examination information registration unit 113 registers the received target in the targets list 128, together with the examinee ID of the instant examinee P and the registration date and time (current time) (STEP 314/FIG. 6B).

It should be noted that the examination information registration unit 113 may skip STEPs 312 to 313 and STEPs 338 to 340, and register a candidate for target received from the doctor terminal 2 of the attending doctor in the targets list, together with the examinee ID of the instant examinee P and the registration date and time. Further, the examination information registration unit 113 may skip STEPs 312 to 313 and STEP 338, and register a target received from the first user terminal 3 of the instant examinee P in the targets list 128, together with the examinee ID of the instant examinee P and the registration date and time.

Further, the examination information registration unit 113 may register a target that is derived from the disease name and conditions of the instant examinee on the basis of the information stored in the server storage unit 12, as a target of the instant examinee P in the targets list 128, together with the examinee ID of the instant examinee P and the registration date and time. For example, assume that the name of the disease of the instant examinee P specified from the examinee list 121 or the medical record information is "hypertension", that the blood pressure of the instant examinee P specified from the medical record information is "160 over 100", and that the desired blood pressure stored in the server storage unit 12 is "130 over 70". In this case, the examination information registration unit 113 may specify a target for the disease name "hypertension" as one related to "blood pressure" on the basis of a correspondence table (not shown), for example, and register the blood pressure "130 over 70" as the target of the instant examinee P in the targets list 128, together with the examinee ID of the instant examinee P and the registration date and time. Alternatively, on the basis of an index of "10" as a blood pressure drop in a certain period stored in the server storage unit 12, the examination information registration unit 113 may register "150 over 90" as the target of the instant examinee in the targets list 128, together with the examinee ID of the instant examinee P and the registration date and time. Furthermore, the examination information registration unit 113 may register wearing of wearable equipment or a target action associated with the disease name "hypertension" on the basis of a correspondence table (not shown), as the target of the instant examinee P in the targets list 128, together with the examinee ID of the instant examinee P and the registration date and time.

This completes the remote diagnostic aid processing.

(Medical Expense Billing and Remuneration Paying Processing)

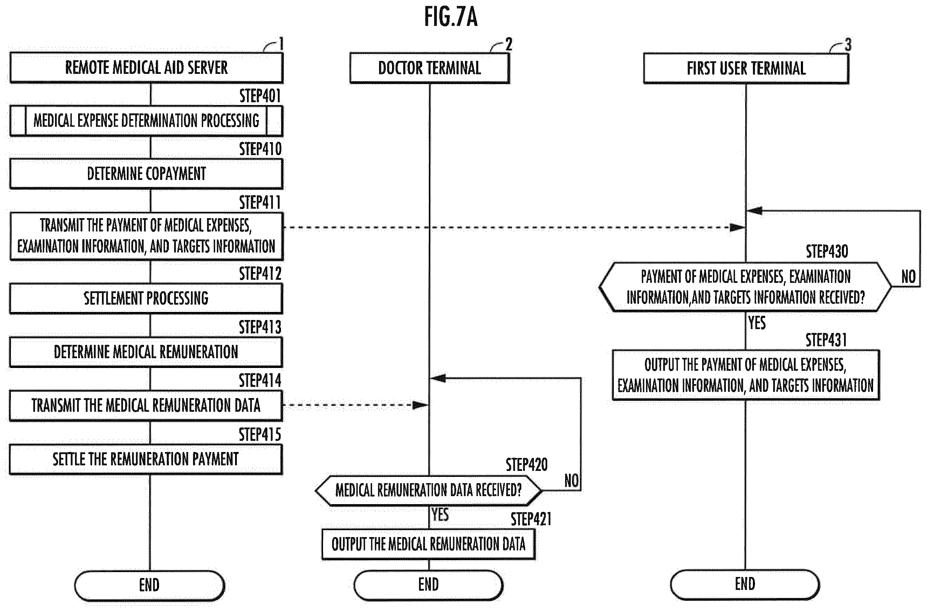

The medical expense billing and remuneration paying processing will be described with reference to FIG. 7A.

The medical expense determination unit 114 performs medical expense determination processing (described later) to determine medical expenses of the instant examinee P (STEP 401/FIG. 7A). In the medical expense determination processing, the medical expense determination unit 114 recognizes targets information which includes: a next target, a current target, an importance level of the next target, an importance level of the current target, an adherence/achievement level of the current target, points this time, and cumulative points.

The medical expense determination unit 114 determines a copayment of medical expenses of the instant examinee P, on the basis of the medical expenses determined in STEP 401/FIG. 7A and the copayment rate included in the examinee list (STEP 410/FIG. 7A).

The medical expense determination unit 114 transmits, to the first user terminal 3 via the server communication unit 15, the determined copayment of medical expenses of the instant examinee P, the examination information of the instant examinee P, and the targets information read from the targets list 128 and the condition log 129 (STEP 411/FIG. 7A). The first user terminal control unit 31 determines whether those items of information have been received via the first user terminal communication unit 35 (STEP 430/FIG. 7A).

If the determination result is negative ("NO" in STEP 430/FIG. 7A), the first user terminal control unit 31 performs the process in STEP 430/FIG. 7A again.

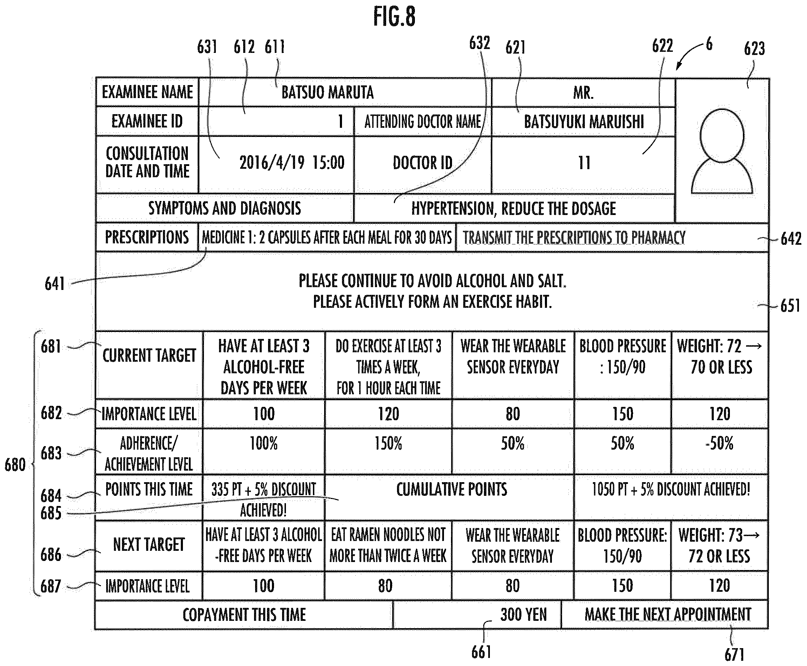

If the determination result is positive ("YES" in STEP 430/FIG. 7A), the first user terminal control unit 31 outputs an examination information screen 6 including the received items of information, as shown in FIG. 8, to the first user terminal output unit 33 (STEP 431/FIG. 7A).

The examination information screen 6 is configured as a Web screen, for example, and includes: an examinee name display 611, an examinee ID display 612, an attending doctor name display 621, a doctor ID display 622 of the attending doctor, a photograph display 623 of the attending doctor, a consultation date and time display 631, a symptoms and diagnosis display 632, a prescriptions display 641, a transmit the prescriptions display 642, a doctor's comment 651, a copayment of medical expenses this time display 661, a next appointment display 671, and a targets display 680.

The targets display 680 includes: a current target display 681, a current target's importance level display 682, a current target's adherence/achievement level display 683, a points this time display 684, a cumulative points display 685, a next target display 686, and a next target's importance level display 687.

In the case where the medical expenses can be discounted, the points this time display 684 and the cumulative points display 685 may each include a corresponding discount display.

In FIG. 8, the points this time display 684 includes a display of 5% discount according to the points this time, and the cumulative points display 685 includes a display of 5% discount according to the cumulative points.

When detecting a click on the transmit the prescriptions display 642 via the first user terminal input unit 34, the first user terminal control unit 31 displays a screen for transmitting uneditable prescriptions to a pharmacy via e-mail or the like. Additionally or alternatively, the first user terminal control unit 31 may transmit a file including the prescriptions with an electronic signature to the first user terminal 3. When detecting a click on the next appointment display 671 via the first user terminal input unit 34, the first user terminal control unit 31 displays an appointment acceptance screen and performs the processes in STEP 130/FIG. 4 and on.

Following the STEP 411/FIG. 7A, the medical expense determination unit 114 uses settlement information of the instant examinee P included in the examinee list 121 to make settlement of the copayment of medical expenses of the instant examinee P (STEP 412/FIG. 7A). For example, the medical expense determination unit 114 transmits the credit information included in the settlement information of the instant examinee P and the copayment of medical expenses to a settlement server specified from the credit information, for settlement of the copayment of medical expenses.

Further, the medical expense determination unit 114 uses the medical expenses and a predetermined formula to determine medical remuneration for the attending doctor D (STEP 413/FIG. 7A).

The medical expense determination unit 114 transmits medical remuneration data to the doctor terminal 2 via the server communication unit 15 (STEP 414/FIG. 7A).

The medical expense determination unit 114 uses the remuneration destination information of the attending doctor D included in the doctor list 122 to settle the payment of the remuneration to the attending doctor D (STEP 415/FIG.7A). For example, the medical expense determination unit 114 transmits the account information of the attending doctor D and the medical remuneration to a bank server specified from the account information, for settlement of the payment of the remuneration to the attending doctor D.

Further, the doctor terminal control unit 21 determines whether medical remuneration data has been received via the doctor terminal communication unit 25 (STEP 420/FIG. 7A).

If the determination result is negative ("NO" in STEP 420/FIG. 7A), the doctor terminal control unit 21 performs the process in STEP 420/FIG. 7A again.

If the determination result is positive ("YES" in STEP 420/FIG. 7A), the doctor terminal control unit 21 outputs a medical remuneration screen, including the received medical remuneration data, to the doctor terminal output unit 23 (STEP 421/FIG. 7A).

This completes the medical expense billing and remuneration paying processing.

(Medical Expense Determination Processing)

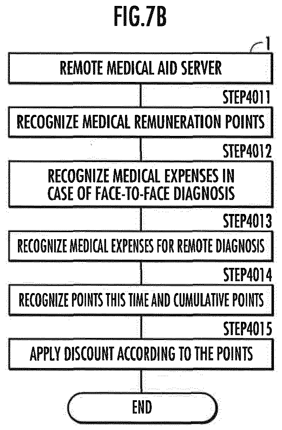

The medical expense determination processing will now be described with reference to FIG. 7B.

The medical expense determination unit 114, on the basis of the examinee ID of the instant examinee P and the current date and time, refers to the examination information list 125 to recognize medical remuneration points this time (STEP 4011/FIG. 7B).

The medical expense determination unit 114 recognizes medical expenses in a case of face-to-face diagnosis, on the basis of the medical remuneration points this time (STEP 4012/FIG. 7B). For example, the medical expense determination unit 114 recognizes, as the medical expenses in the case of face-to-face diagnosis, medical expenses in a general insurance system for a face-to-face diagnosis and for the medical remuneration points this time.

The medical expense determination unit 114, on the basis of the medical remuneration points this time, recognizes medical expenses in a case of remote diagnosis such that they become lower than the medical expenses in the case of face-to-face diagnosis (STEP 4013/FIG. 7B). For example, the medical expense determination unit 114 may calculate the medical expenses in the case of remote diagnosis by multiplying the medical expenses in the case of face-to-face diagnosis by a predetermined positive value smaller than 1, or may calculate the medical expenses in the case of remote diagnosis by deducting a predetermined amount from the medical expenses in the case of face-to-face diagnosis.

The medical expense determination unit 114, on the basis of the examinee ID of the instant examinee P and the current date and time, refers to the targets list 128 to recognize the adherence/achievement level of the current target and the importance level of the current target, and from these, recognizes the points this time and the cumulative points (STEP 4014/FIG. 7B). The medical expense determination unit 114 recognizes the target with the latest registration date as a next target and the target with the second latest registration date as the current target.

The points this time are determined so that the points become higher with increased adherence/achievement level of the current target. For example, the points this time may be calculated by the following expression (1).

[Expression 1]

$$TP = \sum PI*LA \qquad \cdot \cdot (1)$$

Here, TP represents the points this time, PI represents the importance level of the current target, and LA represents the adherence/achievement level (in %) of the current target.

The cumulative points may be a cumulative total of the points of the respective times stored in the server storage unit 12, or a value calculated by using a formula similar to that for the points this time.

The medical expense determination unit 114 applies a discount according to the points this time or the cumulative points, to reduce the medical expenses (STEP 4015/FIG. 7B).

The medical expense determination unit 114 may apply a predetermined discount when a first stipulation that the points this time become not lower than a first point threshold value is fulfilled. Further, the medical expense determination unit 114 may apply a predetermined discount when a second stipulation that the cumulative points become not lower than a second point threshold value is fulfilled. The medical expense determination unit 114 may apply a predetermined discount when a third stipulation that a state where the points this time or the cumulative points are not lower than a third point threshold value has continued for a predetermined period is fulfilled. Further, besides the discounts according to the points this time or the cumulative points, the medical expense determination unit 114 may apply a discount when a fourth stipulation that the adherence/achievement level of a particular target becomes a predetermined adherence/achievement level or higher is fulfilled. Further, the medical expense determination unit 114 may apply a discount when a fifth stipulation that a state where the adherence/achievement level of a particular target is not lower than a predetermined level has continued for a predetermined period is fulfilled.

The medical expense determination unit 114 may reduce, as a discount, a certain rate (for example, 5%) according to the points this time or the cumulative points, or a certain amount (for example, 500 yen) according to the points this time or the cumulative points. After applying a discount, the medical expense determination unit 114 may subtract predetermined points from the cumulative points and store the cumulative points or a notification of such subtraction in the server storage unit 12.

In this manner, the medical expenses are determined appropriately.

(Organization's Payment Determination Processing)

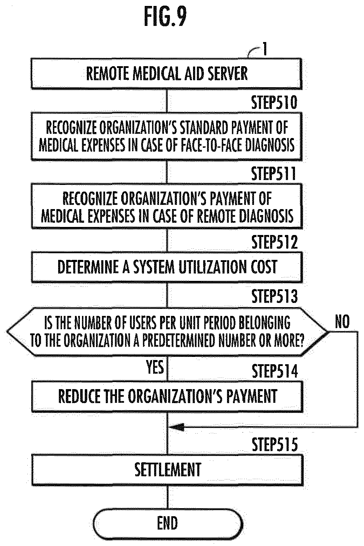

The organization's payment determination processing will be described with reference to FIG. 9.

The organization's payment determination unit 115 calculates an organization's share which is a payment rate of the organization to which the instant examinee P belongs, from the copayment rate of the instant examinee P included in the examinee list 121, and recognizes an amount obtained by multiplying the medical expenses in the case of face-to-face diagnosis, calculated in STEP 4012/FIG. 7B, by the organization's share, as an organization's standard payment of medical expenses which is an amount to be paid by the organization in the case of face-to-face diagnosis (STEP 510/FIG. 9).

The organization's payment determination unit 115 recognizes an amount obtained by multiplying the medical expenses in the remote diagnosis, calculated in STEP 4013/FIG. 7B, by the organization's share, as an organization's payment of medical expenses which is an amount to be paid by the organization in the case of remote diagnosis (STEP 511/FIG. 9).

The organization's payment determination unit 115 determines, as a system utilization cost, a portion of the difference between the organization's standard payment of medical expenses and the organization's payment of medical expenses in the case of remote diagnosis (STEP 512/FIG. 9). For example, the organization's payment determination unit 115 determines a half of the difference between the organization's standard payment of medical expenses and the organization's payment of medical expenses for the instant examinee P as the system utilization cost.

The organization's payment determination unit 115 determines whether the number of users per unit period belonging to the organization is a predetermined number or higher (STEP 513/FIG. 9). For example, the organization's payment determination unit 115 refers to the examinee list 121 to recognize persons who belong to the same organization as the instant examinee P. The organization's payment determination unit 115 then recognizes how many of those recognized persons have utilized the remote diagnostic system during a unit period of "one month" up to the current time. The organization's payment determination unit 115 determines whether the number of such users is the predetermined number or higher.

If the determination result is positive ("YES" in STEP 513/FIG. 9), the organization's payment determination unit 115 reduces the organization's payment of medical expenses as well as the system utilization cost (STEP 514/FIG. 9).

Following the process in STEP 514/FIG. 9, or if the determination result in STEP 513/FIG. 9 is negative ("NO" in STEP 513/FIG. 9), the organization's payment determination unit 115 recognizes the organization ID of the instant examinee P included in the examinee list 121, and uses the settlement information of the organization specified from the organization ID included in the organization list 123, to make settlement of a sum of the organization's payment of medical expenses and the determined system utilization cost (STEP 515/FIG. 9).

This completes the organization's payment determination processing.

(Processing of Providing Data on Organization's Payment of Medical Expenses)

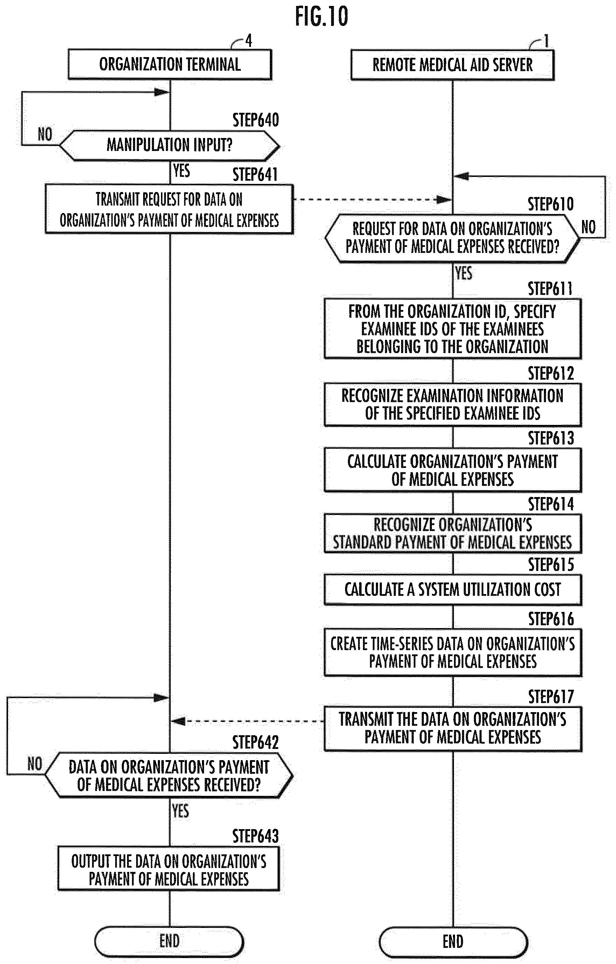

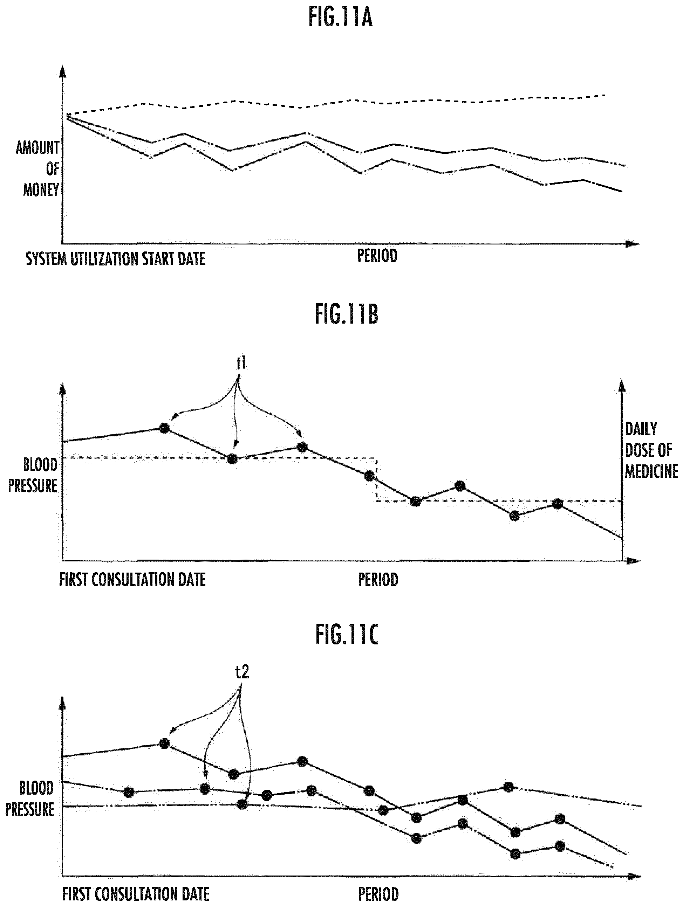

Now, referring to FIGs. 10 and 11A, processing of providing data on organization's payment of medical expenses will be described, in which time-series data on organization's payment of medical expenses for an organization is provided to a person W in charge in the organization.

The organization terminal control unit 41 determines whether a manipulation of requesting display of data on the organization's payment of medical expenses has been input via the organization terminal input unit 44 (STEP 640/FIG. 10).

If the determination result is negative ("NO" in STEP 640/FIG. 10), the organization terminal control unit 41 performs the process in STEP 640/FIG. 10 again.

If the determination result is positive ("YES" in STEP 640/FIG. 10), the organization terminal control unit 41 transmits to the remote diagnostic aid server 1, via the organization terminal communication unit 45, a request for data on the organization's payment of medical expenses including the organization ID stored in the organization terminal storage unit 42 (STEP 641/FIG. 10).

The data transmission unit 116 determines whether a request for data on the organization's payment of medical expenses has been received via the server communication unit 15 (STEP 610/FIG. 10).

If the determination result is negative ("NO" in STEP 610/FIG. 10), the data transmission unit 116 performs the process in STEP 610/FIG. 10 again.

If the determination result is positive ("YES" in STEP 610/FIG. 10), the data transmission unit 116, on the basis of the organization ID included in the request for the data on the organization's payment of medical expenses, refers to the examinee list 121 to specify an examinee ID of any examinee belonging to that organization (STEP 611/FIG. 10). For example, from the organization ID "101" included in the request for the data on the organization's payment of medical expenses from the organization "Company A", the data transmission unit 116 refers to the examinee list 121 shown in FIG. 2A to thereby specify examinee IDs "1" and "3" of "Batsuo MARUTA" and "Marumi BATSUGAWA" having their member numbers including the organization ID "101" at the head.

The data transmission unit 116 refers to the examination information list 125 to recognize examination information including the specified examinee IDs (STEP 612/FIG. 10).

For example, from the examination information list 125 shown in FIG. 2E, the data transmission unit 116 extracts and recognizes the fields with the examinee IDs of "1" and "3".

The data transmission unit 116 calculates an organization's payment of medical expenses for each unit period, on the basis of the recognized examination information (STEP 613/FIG. 10).

For example, the data transmission unit 116 sums up the organization's payments of medical expenses, recognized similarly as in STEP 511/FIG. 9, in unit periods of "one month", to recognize the organization's payment of medical expenses for each unit period of "one month".

The data transmission unit 116 recognizes, on the basis of the recognized examination information, an organization's standard payment of medical expenses for each unit period of that organization (STEP 614/FIG. 10). For example, the data transmission unit 116 sums up the organization's standard payments of medical expenses, recognized in a similar manner as in STEP 510/FIG. 9, in unit periods of "one month", to recognize the organization's standard payment of medical expenses for each unit period of "one month". The data transmission unit 116 calculates a portion of the difference between the organization's standard payment of medical expenses for each unit period and the organization's payment of medical expenses for each unit period, as a system utilization cost for each unit period (STEP 615/FIG. 10).

The data transmission unit 116 creates time-series data on the organization's payment of medical expenses, on the basis of the organization's standard payment of medical expenses for each unit period, the organization's payment of medical expenses for each unit period, and the system utilization cost for each unit period (STEP 616/FIG. 10).

FIG. 11A is a line graph in time series order of the organization's payment of medical expenses included in the data on the organization's payment of medical expenses. In FIG. 11A, the vertical axis represents amount of money, and the horizontal axis represents period. The broken line is a graph showing a transition of the organization's standard payment of medical expenses, the dot-and-dash line is a graph showing a transition of the organization's payment of medical expenses, and a two-dot chain line is a graph showing a transition of the amount obtained by adding the system utilization cost to the organization's payment of medical expenses.

The data transmission unit 116 transmits the created data on the organization's payment of medical expenses to the organization terminal 4 via the server communication unit 15 (STEP 617/FIG. 10).

The organization terminal control unit 41 determines whether data on the organization's payment of medical expenses has been received via the organization terminal communication unit 45 (STEP 642/FIG. 10).

If the determination result is negative ("NO" in STEP 642/FIG. 10), the organization terminal control unit 41 performs the process in STEP 642/FIG. 10 again.

If the determination result is positive ("YES" in STEP 642/FIG. 10), the organization terminal control unit 41 outputs the received data on the organization's payment of medical expenses to the organization terminal output unit 43, in the form of a graph as shown in FIG. 11A, for example (STEP 643/FIG. 10).

This completes the processing of providing the data on the organization's payment of medical expenses.

(Processing of Providing First Health Condition Data)

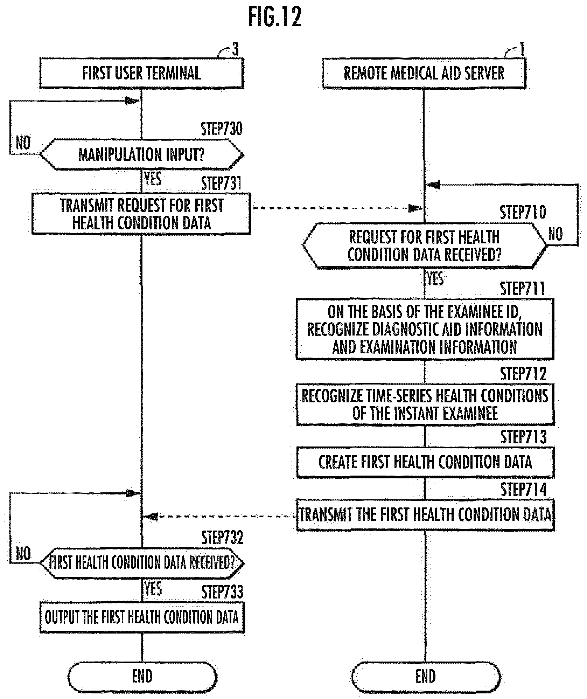

Referring to FIGs. 11B and 12, a description will be made about processing of providing first health condition data, or, processing of providing data indicating time-series changes of health conditions of an instant examinee P to the first user terminal 3 of the instant examinee P.

The first user terminal control unit 31 determines whether a manipulation of requesting display of first health condition data has been input via the first user terminal input unit 34 (STEP 730/FIG. 12).

If the determination result is negative ("NO" in STEP 730/FIG. 12), the first user terminal control unit 31 performs the process in STEP 730/FIG. 12 again.

If the determination result is positive ("YES" in STEP 730/FIG. 12), the first user terminal control unit 31 transmits a request for the first health condition data, including the examinee ID of the instant examinee P stored in the first user terminal storage unit 32, to the remote diagnostic aid server 1 via the first user terminal communication unit 35 (STEP 731/FIG. 12).

The data transmission unit 116 determines whether a request for the first health condition data has been received via the server communication unit 15 (STEP 710/FIG. 12).

If the determination result is negative ("NO" in STEP 710/FIG. 12), the data transmission unit 116 performs the process in STEP 710/FIG. 12 again.

If the determination result is positive ("YES" in STEP 710/FIG. 12), the data transmission unit 116, on the basis of the examinee ID of the instant examinee P included in the request for the first health condition data, refers to the diagnostic aid information list 124 and the examination information list 125 to recognize diagnostic aid information and examination information including that examinee ID (STEP 711/FIG. 12). It should be noted that even if the data transmission unit 116 receives no request for the first health condition data, the unit may perform the processes in STEP 711/FIG. 12 and on at regular intervals to transmit the first health condition data to the contact address, such as the e-mail address, of the examinee ID of the instant examinee P.

For example, the data transmission unit 116 extracts, from the diagnostic aid information list 124 and the examination information list 125 shown in FIGs. 2D and 2E, the information in the fields with the examinee ID "1" of the instant examinee P, and recognizes the extracted information.

The data transmission unit 116, on the basis of the recognized information, recognizes the health conditions of the instant examinee P in time series at the respective times of consultation as well as the information on the medicines prescribed for the instant examinee P (STEP 712/FIG. 12).

For example, the data transmission unit 116 may refer to the diagnostic aid information list 124 to recognize the blood pressure of the instant examinee P at each time of consultation, and refer to the examination information list 125 to recognize the daily dose of medicine. It should be noted that the health conditions in the diagnostic aid information having the registration date and time closest to the consultation date and time included in the examination information list 125 may be recognized as the health conditions of the instant examinee P at the time of consultation. Further, the item "blood pressure" of health condition to be recognized may be determined, on the basis of the disease name "hypertension" of the instant examinee P in the examinee list 121, by referring to a correspondence table (not shown). Alternatively, the data transmission unit 116 may recognize either one of the health conditions of the instant examinee P in time series at the respective consultation times and the information on the medicines prescribed for the instant examinee P.

The data transmission unit 116 creates time-series first health condition data, on the basis of the health conditions of the instant examinee P in time series at the respective consultation times and the information on the medicines prescribed for the instant examinee P (STEP 713/FIG. 12).

The data transmission unit 116 transmits the created first health condition data to the first user terminal 3 via the server communication unit 15 (STEP 714/FIG. 12).

The first user terminal control unit 31 determines whether first health condition data has been received via the first user terminal communication unit 35 (STEP 732/FIG. 12).

If the determination result is negative ("NO" in STEP 732/FIG. 12), the first user terminal control unit 31 performs the process in STEP 732/FIG. 12 again.

If the determination result is positive ("YES" in STEP 732/FIG. 12), the first user terminal control unit 31 outputs the received first health condition data to the first user terminal output unit 33, in the form of a graph as shown in FIG. 11B, for example (STEP 733/FIG. 12).

FIG. 11B is a graph created on the basis of the first health condition data. In FIG. 11B, the left vertical axis represents blood pressure, the right vertical axis represents daily dose of medicine, and the horizontal axis represents period. The solid line is a graph indicating a transition of blood pressure, and the broken line is a graph indicating a transition of daily dose of medicine. Points t1 in parts of the graphs, each point indicating a time of consultation, include a link to an examination information screen which corresponds to the examination information screen shown in FIG. 8 except that the link on the transmit the prescriptions display has been deleted therefrom.

This completes the processing of providing the first health condition data.

(Processing of Providing Second Health Condition Data)

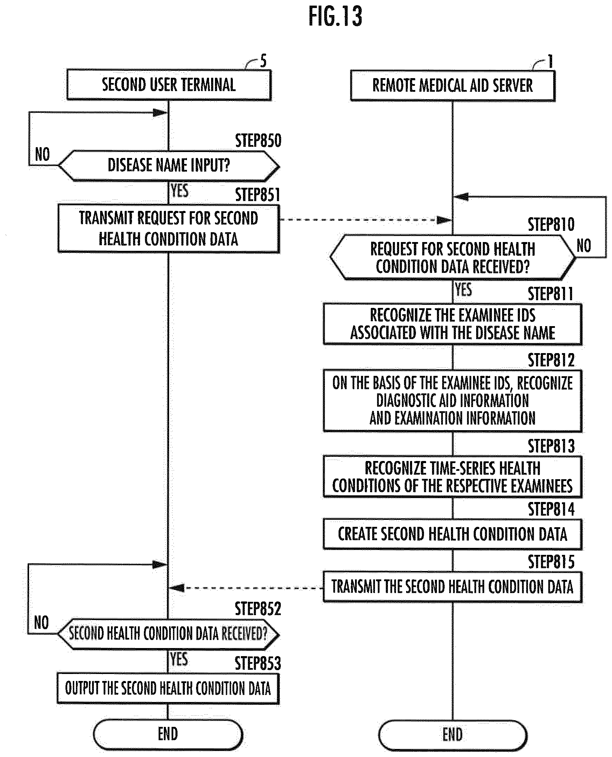

Referring to FIGs. 11C and 13, a description will be made about processing of providing second health condition data, or, processing of providing a second user terminal 5 of a potential examinee UP, who has not utilized the remote diagnostic aid service of the remote diagnostic aid server 1, with second health condition data indicating time-series transitions of health conditions of remote diagnostic aid service users (examinees) related to the disease name designated by the potential examinee UP.

The second user terminal control unit 51 determines whether a manipulation of requesting display of second health condition data including designation of a disease name has been input via the second user terminal input unit 54 (STEP 850/FIG. 13).

If the determination result is negative ("NO" in STEP 850/FIG. 13), the second user terminal control unit 51 performs the process in STEP 850/FIG. 13 again.

If the determination result is positive ("YES" in STEP 850/FIG. 13), the second user terminal control unit 51 transmits a request for the second health condition data, including the designated disease name, to the remote diagnostic aid server 1 via the second user terminal communication unit 55 (STEP 851/FIG. 13).

The data transmission unit 116 determines whether a request for the second health condition data has been received via the server communication unit 15 (STEP 810/FIG. 13).

If the determination result is negative ("NO" in STEP 810/FIG. 13), the data transmission unit 116 performs the process in STEP 810/FIG. 13 again.

If the determination result is positive ("YES" in STEP 810/FIG. 13), the data transmission unit 116 refers to the examinee list 121 to recognize, from the disease name included in the request for the second health condition data, any examinee ID associated with that disease name (STEP 811/FIG. 13).

For example, from the disease name "hypertension" included in the request for the second health condition data, the data transmission unit 116 refers to the examinee list 121 to recognize the examinee ID "1" associated with that disease name "hypertension".

The unit then refers to the diagnostic aid information list 124 and the examination information list 125 to recognize any field including that examinee ID (STEP 812/FIG. 13).

For example, the data transmission unit 116 extracts information in the fields with the examinee ID "1" from the diagnostic aid information list 124 and the examination information list 125 shown in FIGs. 2D and 2E, and recognizes the extracted information.

The data transmission unit 116, on the basis of the recognized information, recognizes health conditions of each examinee in time series at the consultation times and information on medicines prescribed for each examinee (STEP 813/FIG. 13).

For example, the data transmission unit 116 may refer to the diagnostic aid information list 124 to recognize the blood pressure of each examinee at each time of consultation, and refer to the examination information list 125 to recognize the daily dose of medicine. It should be noted that the health conditions in the diagnostic aid information having the registration date and time closest to the consultation date and time included in the examination information list 125 may be recognized as the health conditions of each examinee at the time of consultation. Further, the health condition item "blood pressure" to be recognized may be determined, on the basis of the designated disease name "hypertension", by referring to a correspondence table (not shown). Alternatively, the data transmission unit 116 may recognize either one of the health conditions of each examinee in time series at the respective consultation times and the information on the medicines prescribed for each examinee.

The data transmission unit 116 creates time-series second health condition data on the basis of the health conditions of respective examinees at the respective consultation times and the information on the medicines prescribed for the respective examinees (STEP 814/FIG. 13).

The data transmission unit 116 transmits the created second health condition data to the second user terminal 5 via the server communication unit 15 (STEP 815/FIG. 13).

The second user terminal control unit 51 determines whether second health condition data has been received via the second user terminal communication unit 55 (STEP 852/FIG. 13).

If the determination result is negative ("NO" in STEP 852/FIG. 13), the second user terminal control unit 51 performs the process in STEP 852/FIG. 13 again.

If the determination result is positive ("YES" in STEP 852/FIG. 13), the second user terminal control unit 51 outputs the received second health condition data to the second user terminal output unit 53, in the form of a graph as shown in FIG. 11C, for example (STEP 853/FIG. 13).

FIG. 11C is a line graph showing time-series transitions of the health conditions for respective examinees included in the second health condition data. In FIG. 11C, the left vertical axis represents blood pressure, and the horizontal axis represents period. The graph for each examinee may be a graph showing a transition of health conditions in a predetermined period (of one year, for example) starting from the examinee's initial date of consultation. The solid line, the dot-and-dash line, and the two-dot chain line are graphs showing transitions of blood pressure of the respective examinees. Each point t2 indicating a respective time of consultation in each graph includes a link to an examination information screen which corresponds to the examination information screen shown in FIG. 8 except that personal information such as the examinee names and the examinee IDs, the transmit the prescriptions display, and the appointment acceptance display have been deleted therefrom.

This completes the processing of providing the second health condition data.


(Second Embodiment)

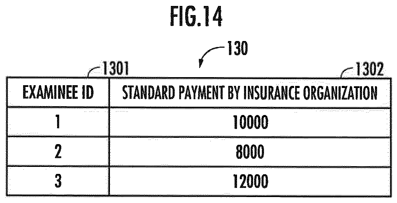

A remote diagnostic aid system according to a second embodiment of the present invention will be described with reference to FIGs. 14 to 16.

The second embodiment corresponds to the remote diagnostic aid system according to the first embodiment except that the organization in the present embodiment is an insurance organization that utilizes a public health care system.

In the description of the second embodiment, the same elements as in the first embodiment are denoted by the same reference numerals and a description thereof will not be repeated. It should be noted that the organization terminal 4 corresponds to the "insurance organization terminal" in the present invention.

In the second embodiment, the member number column 1216 included in the examinee list 121 stores insurance numbers assigned to the respective examinees. The insurance numbers each include an ID for identifying the insurance organization with which the corresponding examinee is enrolled.

In the second embodiment, the server storage unit 12 is configured to further store an insurance organization's standard payment information list 130 and payment facility information 131.

The insurance organization's standard payment information list 130, as shown in FIG. 14, includes an examinee ID column 1301, and an insurance organization's standard payment column 1302.

The examinee ID column 1301 stores examinee IDs of respective examinees. The insurance organization's standard payment column 1302 stores the insurance organizations' standard payments for the respective examinees.

The insurance organization's standard payment for each examinee is an amount to be paid by an insurance organization out of medical expenses (including charges for medicines) in the case of face-to-face diagnosis, which are calculated, for example, from the health conditions of the examinee on the initial date of consultation for that examinee. Alternatively, an insurance organization's payment out of medical expenses per one time of face-to-face diagnosis (for example, average medical expenses) which are calculated from the health conditions of a respective examinee on a respective date of consultation over a predetermined number of consultations from the first consultation may be adopted as the insurance organization's standard payment for each examinee. Still alternatively, information on at least one of gender, age, and disease name of an examinee may be acquired from the examinee list 121, and an insurance organization's payment out of average medical expenses in the case of face-to-face diagnosis of the examinees corresponding to the acquired information may be adopted as the insurance organization's standard payment. Furthermore, preset medical expenses may be set as the insurance organization's standard payment.

The payment facility information 131 is information on an insurance treatment fee payment facility which is to be billed for health insurance treatment fees. Supplementally, in general, a medical institution notifies an insurance treatment fee payment facility of the health insurance treatment fees and the insurance number, and receives a payment of the health insurance treatment fees. The insurance treatment fee payment facility bills an insurance organization specified by the insurance number for the health insurance treatment fees.

(Medical Expense Billing and Remuneration Paying Processing)

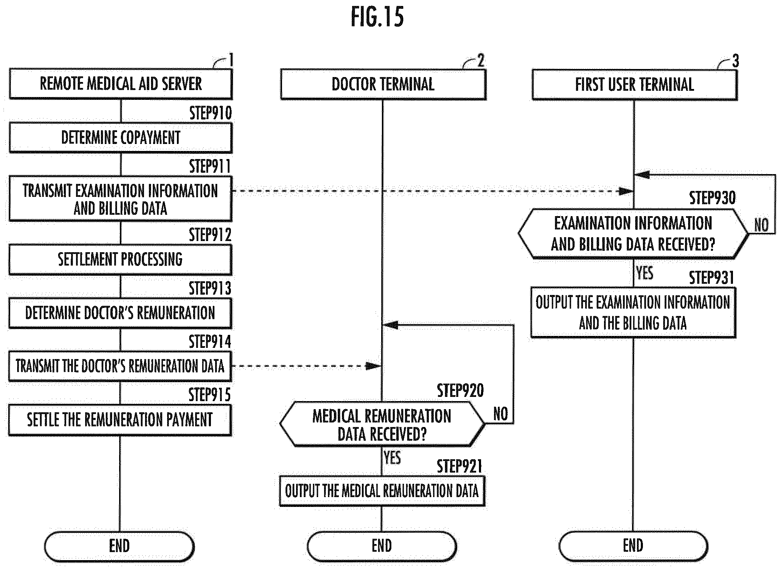

The medical expense billing and remuneration paying processing in the second embodiment will be described with reference to FIG. 15.

The medical expense determination unit 114 determines a copayment of an instant examinee P, by using a predetermined calculation formula, on the basis of medical remuneration points for the instant examinee P included in the examination information list 125 (STEP 910/FIG. 15). Here, the copayment of the instant examinee P is an amount determined on the basis of the medical remuneration points in the remote diagnosis made by the attending doctor D in the remote diagnostic aid processing (STEP 300/FIG. 3); it does not include charges for prescribed medicines.

The medical expense determination unit 114 transmits billing data, including the determined copayment of the instant examinee P and the examination information of the instant examinee P, to the first user terminal 3 via the server communication unit 15 (STEP 911/FIG. 15).

The first user terminal control unit 31 determines whether billing data has been received via the first user terminal communication unit 35 (STEP 930/FIG. 15).

If the determination result is negative ("NO" in STEP 930/FIG. 15), the first user terminal control unit 31 performs the process in STEP 930/FIG. 15 again.

If the determination result is positive ("YES" in STEP 930/FIG. 15), the first user terminal control unit 31 outputs an examination information screen 6, including the received examination information and billing data, to the first user terminal output unit 33 (STEP 931/FIG. 15).

Following the STEP 911/FIG. 15, the medical expense determination unit 114 uses the settlement information of the instant examinee P included in the examinee list 121 to make settlement of the copayment of the instant examinee P (STEP 912/FIG. 15). For example, the medical expense determination unit 114 transmits the copayment and the credit information included in the settlement information of the instant examinee P to a settlement server specified from the credit information, for settlement of the copayment.

Further, the medical expense determination unit 114 determines medical remuneration for the attending doctor D by using the medical remuneration points and a predetermined formula (STEP 913/FIG. 15).

The medical expense determination unit 114 transmits the medical remuneration data to the doctor terminal 2 via the server communication unit 15 (STEP 914/FIG. 15).

The medical expense determination unit 114 uses the remuneration destination information of the attending doctor D included in the doctor list 122 to settle the payment of remuneration to the attending doctor D (STEP 915/FIG. 15). For example, the medical expense determination unit 114 transmits the medical remuneration and the account information of the attending doctor D to a bank server specified from the account information, for settlement of the payment of the remuneration to the attending doctor D.

The doctor terminal control unit 21 determines whether medical remuneration data has been received via the doctor terminal communication unit 25 (STEP 920/FIG. 15).

If the determination result is negative ("NO" in STEP 920/FIG. 15), the doctor terminal control unit 21 performs the process in STEP 920/FIG. 15 again.

If the determination result is positive ("YES" in STEP 920/FIG. 15), the doctor terminal control unit 21 outputs a medical remuneration screen, including the received medical remuneration data, to the doctor terminal output unit 23 (STEP 921/FIG. 15).

This completes the medical expense billing and remuneration paying processing.

(Organization's Payment Determination Processing)

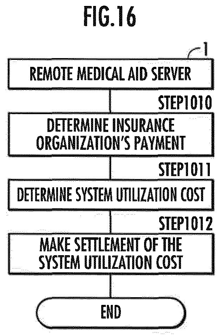

The organization's payment determination processing in the second embodiment will be described with reference to FIG. 16.

The organization's payment determination unit 115, on the basis of the examination information of the instant examinee P included in the examination information list 125 and the copayment rate of the instant examinee P included in the examinee list 121, calculates an insurance organization's payment which is an amount to be paid by the insurance organization with which the instant examinee P is enrolled, out of the medical expenses (STEP 1010/FIG. 16). Here, the insurance organization's payment is an amount obtained by summing up the health insurance treatment fees, temporarily paid by the insurance treatment fee payment facility, and an insurance organization's payment out of charges for medicines which are specified from the prescriptions included in the examination information.

For example, assuming that the copayment rate of the instant examinee P is BR, the medical remuneration points included in the examination information are W, the rate is X yen per medical remuneration point, Medicine 1 has been prescribed Y capsules in total, and the unit cost of the Medicine 1 acquired from an external medicine price server is Z yen per capsule, then the organization's payment determination unit 115 calculates the insurance organization's payment IB by the following expression (2).

[Expression 2]

$$IB = (W * X + Y * Z) * (1 - BR) \qquad \cdot (2)$$

The organization's payment determination unit 115 determines, as a system utilization cost, a portion of the difference between the insurance organization's standard payment for the instant examinee P, shown in the insurance organization's standard payment information list 130, and the insurance organization's payment (STEP 1011/ FIG. 16). For example, the organization's payment determination unit 115 determines a half of the difference between the insurance organization's standard payment and the insurance organization's payment for the instant examinee P as the system utilization cost.

The organization's payment determination unit 115 recognizes the insurance organization ID of the instant examinee P included in the examinee list 121, and uses the settlement information of the insurance organization specified from the insurance organization ID included in the insurance organization list 123, to make settlement of the determined system utilization cost (STEP 1012/ FIG.16).

This completes the organization's payment determination processing.

Description of Reference Numerals

1: remote diagnostic aid server; 2: doctor terminal; 3: first user terminal (user terminal); 4: organization terminal; 111: consultation appointment acceptance unit; 112: diagnostic aid information registration unit; 113: examination information registration unit; 114: medical expense determination unit; 115: organization's payment determination unit; 116: data transmission unit; 12: server storage unit; 121: examinee list; 122: doctor list; 123: organization list; 124: diagnostic aid information list; 125: examination information list; 126: appointment list; 127: available consultation time information list; 128: targets list; 129: condition log; and 21: doctor terminal control unit (remote diagnostic aid unit).

**Claims**

**1.** A remote diagnostic aid system being a network system including a remote diagnostic aid server and one or more doctor terminals communicating with a plurality of user terminals, the remote diagnostic aid system comprising:

a storage unit which stores available consultation time information including an available consultation time during which a doctor as a user of a respective one of the one or more doctor terminals is available for consultation, and payment rate information indicating, for a respective one of users of the plurality of user terminals, a copayment rate which is a payment rate of the user and an organization's share of medical expenses which is a payment rate of an organization to which the user belongs;

a consultation appointment acceptance unit which receives, from a first user terminal as one of the plurality of user terminals, information including a desired consultation time during which an instant examinee as a user of the first user terminal wants to have a consultation, refers to the available consultation time information to extract an attending doctor who is available for consultation during the desired consultation time, and transmits, to one or both of the first user terminal and an attending doctor terminal as the doctor terminal of the attending doctor, communication establishment information for establishing a communication between the attending doctor terminal and the first user terminal;

a remote diagnostic aid unit which outputs examinee's statement information, received from the first user terminal with which the communication has been established on the basis of the communication establishment information, to the attending doctor terminal, and transmits doctor's statement information, input from the attending doctor terminal, to the first user terminal;

a medical expense determination unit which determines medical expenses for the instant examinee, on the basis of medical record information of the instant examinee received from the attending doctor terminal or the first user terminal, such that the medical expenses become lower than standard medical expenses for the same content of diagnosis in a case of face-to-face diagnosis; and

an organization's payment determination unit which determines, as a system utilization cost, a portion of a difference between an organization's standard payment of medical expenses, obtained by multiplying the standard medical expenses by the organization's share of medical expenses of an organization to which the instant examinee belongs, and an organization's payment of medical expenses, obtained by multiplying the medical expenses by the organization's share of medical expenses.

2. The remote diagnostic aid system according to claim 1, wherein

the storage unit stores information on an organization's payment of medical expenses in which a consultation time point, an examinee, and an organization's payment of medical expenses are associated with one another, and

the remote diagnostic aid system is configured to comprise a data transmission unit which refers to the information on the organization's payment of medical expenses to transmit, to a terminal of an organization, data on an organization's payment of medical expenses indicating a relationship between the consultation time point and the organization's payment of medical expenses for an examinee associated with the organization.

3. The remote diagnostic aid system according to claim 2, wherein

the storage unit stores information on an organization's standard payment of medical expenses in which a consultation time point, an examinee, and an organization's standard payment of medical expenses are associated with one another, and

the remote diagnostic aid system is configured to comprise a data transmission unit which refers to the information on the organization's standard payment of medical expenses to transmit, to a terminal of an organization, data on an organization's standard payment of medical expenses indicating a relationship between the consultation time point and the organization's standard payment of medical expenses for an examinee associated with the organization, together with the data on the organization's payment of medical expenses.

4. The remote diagnostic aid system according to any one of claims 1 to 3, wherein

the storage unit stores medical record information in which a consultation time point, an examinee, and one or both of health condition information of the examinee and information on medicines prescribed for the examinee are associated with one another, and

the remote diagnostic aid system is configured to comprise a data transmission unit which refers to the medical record information to transmit, to a user terminal of an examinee, first health condition data associated with the examinee indicating a relationship between the consultation time point and one or both of the health condition information and the information on the medicines prescribed for the examinee.

5. The remote diagnostic aid system according to any one of claims 1 to 4, wherein

the storage unit stores medical record information in which a consultation time point, and one or both of health condition information and information on prescribed medicines including a disease name are associated with each other, and

the remote diagnostic aid system is configured to comprise a data transmission unit which

receives, from a second user terminal as one of the user terminals, a request for health condition information including a disease name, and refers to the medical record information to extract one or both of the health condition information and the information on the prescribed medicines including the received disease name, and

transmits, to the second user terminal, second health condition data indicating time-series transitions of one or both of the extracted health condition information and the extracted information on the prescribed medicines.

**6.** A remote diagnostic aid server communicating with a plurality of user terminals and one or more doctor terminals, the remote diagnostic aid server comprising:

a storage unit which stores available consultation time information including an available consultation time during which a doctor as a user of a respective one of the one or more doctor terminals is available for consultation, and payment rate information indicating, for a respective one of users of the plurality of user terminals, a copayment rate which is a payment rate of the user and an organization's share of medical expenses which is a payment rate of an organization to which the user belongs;

a consultation appointment acceptance unit which receives, from a first user terminal as one of the plurality of user terminals, information including a desired consultation time during which an instant examinee as a user of the first user terminal wants to have a consultation, refers to the available consultation time information to extract an attending doctor who is available for consultation during the desired consultation time, and transmits, to one or both of the first user terminal and an attending doctor terminal as the doctor terminal of the attending doctor, communication establishment information for establishing a communication between the attending doctor terminal and the first user terminal;

a medical expense determination unit which determines medical expenses for the instant examinee, on the basis of medical record information of the instant examinee received from the attending doctor terminal or the first user terminal, such that the medical expenses become lower than standard medical expenses for the same content of diagnosis in a case of face-to-face diagnosis; and

an organization's payment determination unit which determines, as a system utilization cost, a portion of a difference between an organization's standard payment of medical expenses, obtained by multiplying the standard medical expenses by the organization's share of medical expenses of an organization to which the instant examinee belongs, and an organization's payment of medical expenses, obtained by multiplying the medical expenses by the organization's share of medical expenses.

**7.** A remote diagnostic aid method performed by a remote diagnostic aid server, the remote diagnostic aid server communicating with a plurality of user terminals and one or more doctor terminals, and including a storage unit which stores available consultation time information including an available consultation time during which a doctor as a user of a respective one of the one or more doctor terminals is available for consultation, and payment rate information indicating, for a respective one of users of the plurality of user terminals, a copayment rate which is a payment rate of the user and an organization's share of medical expenses which is a payment rate of an organization to which the user belongs, the method comprising:

a step of receiving, from a first user terminal as one of the plurality of user terminals, information including a desired consultation time during which an instant examinee as a user of the first user terminal wants to have a consultation;

a step of referring to the available consultation time information to extract an attending doctor who is available for consultation during the desired consultation time;

a step of transmitting, to one or both of the first user terminal and an attending doctor terminal as the doctor terminal of the attending doctor, communication establishment information for establishing a communication between the attending doctor terminal and the first user terminal;

a step of determining medical expenses for the instant examinee, on the basis of medical record information of the instant examinee received from the attending doctor terminal or the first user terminal, such that the medical expenses become lower than standard medical expenses for the same content of diagnosis in a case of face-to-face diagnosis; and

a step of determining, as a system utilization cost, a portion of a difference between an organization's standard payment of medical expenses, obtained by multiplying the standard medical expenses by the organization's share of medical expenses of an organization to which the instant examinee belongs, and an organization's payment of medical expenses, obtained by multiplying the medical expenses by the organization's share of medical expenses.

**8.** A remote diagnostic aid system being a network system including a remote diagnostic aid server and one or more doctor terminals communicating with a plurality of user terminals, the remote diagnostic aid system comprising:

a storage unit which stores available consultation time information including an available consultation time during which a doctor associated with a respective one of the one or more doctor terminals is available for consultation, and information on an insurance organization's standard payment which is a standard amount to be paid by an insurance organization;

a consultation appointment acceptance unit which receives, from a first user terminal as one of the plurality of user terminals, a desired consultation time during which an instant examinee associated with the first user terminal wants to have a consultation, refers to the available consultation time information to extract an attending doctor who is available for consultation during the desired consultation time, and transmits, to one or both of the first user terminal and an attending doctor terminal as the doctor terminal of the attending doctor, communication establishment information for establishing a communication between the attending doctor terminal and the first user terminal;

a remote diagnostic aid unit which outputs examinee's statement information, received from the first user terminal with which the communication has been established on the basis of the communication establishment information, to the attending doctor terminal, and transmits doctor's statement information, input from the attending doctor terminal, to the first user terminal; and

a system utilization cost settlement unit which, on the basis of medical record information of the instant examinee received from the attending doctor terminal or the first user terminal, calculates a part or whole of medical expenses for the instant examinee as an insurance organization's payment which is to be paid by an insurance organization with which the instant examinee is enrolled, and uses settlement information of the insurance organization to make settlement of a portion of a difference between the calculated insurance organization's payment and the insurance organization's standard payment as a system utilization cost.

9. A remote diagnostic aid method, performed by one of a remote diagnostic aid server and one or more doctor terminals, or performed through cooperation of the remote diagnostic aid server and the one or more doctor terminals, each communicating with a plurality of user terminals and including a storage unit which stores available consultation time information, including an available consultation time during which a doctor associated with a respective one of the one or more doctor terminals is available for consultation, and information on an insurance organization's standard payment which is a standard amount to be paid by an insurance organization, the method comprising:

a consultation appointment accepting step of receiving, from a first user terminal as one of the plurality of user terminals, a desired consultation time during which an instant examinee associated with the first user terminal wants to have a consultation, referring to the available consultation time information to extract an attending doctor who is available for consultation during the desired consultation time, and transmitting, to one or both of the first user terminal and an attending doctor terminal as the doctor terminal of the attending doctor, communication establishment information for establishing a communication between the attending doctor terminal and the first user terminal; and

a system utilization cost settlement step of, on the basis of medical record information of the instant examinee received from the attending doctor terminal or the first user terminal, calculating a part or whole of medical expenses for the instant examinee as an insurance organization's payment which is to be paid by an insurance organization with which the instant examinee is enrolled, and using settlement information of the insurance organization to make settlement of a portion of a difference between the calculated insurance organization's payment and the insurance organization's standard payment as a system utilization cost.

10. A remote diagnostic aid system being a network system including a remote diagnostic aid server and one or more doctor terminals communicating with a plurality of user terminals, the remote diagnostic aid system comprising:

a storage unit which stores available consultation time information including an available consultation time during which a doctor as a user of a respective one of the one or more doctor terminals is available for consultation;

a consultation appointment acceptance unit which receives, from a first user terminal as one of the plurality of user terminals, information including a desired consultation time during which an instant examinee as a user of the first user terminal wants to have a consultation, refers to the available consultation time information to extract an attending doctor who is available for consultation during the desired consultation time, and transmits, to one or both of the first user terminal and an attending doctor terminal as the doctor terminal of the attending doctor, communication establishment information for establishing a communication between the attending doctor terminal and the first user terminal;

a remote diagnostic aid unit which outputs examinee's statement information, received from the first user terminal with which the communication has been established on the basis of the communication establishment information, to the attending doctor terminal, and transmits doctor's statement information, input from the attending doctor terminal, to the first user terminal; and

a medical expense determination unit which recognizes a condition of the instant examinee on the basis of medical record information received from the attending doctor terminal or the first user terminal, determines whether the condition of the instant examinee fulfills a current stipulation, determines medical expenses for the instant examinee such that the medical expenses when it is determined that the condition of the instant examinee fulfills the current stipulation become not higher than the medical expenses when it is determined that the condition of the instant examinee fails to fulfill the current stipulation, recognizes information indicating a next stipulation on the basis of the medical record information, and transmits the medical expenses, whether the current stipulation has been fulfilled or not, and the information indicating the next stipulation to the first user terminal.

11. The remote diagnostic aid system according to claim 10, wherein
the stipulation is that the instant examinee is using a predetermined wearable device which automatically acquires a health condition of the instant examinee for transmission to the remote diagnostic aid system.

12. The remote diagnostic aid system according to claim 10 or 11, wherein
the stipulation is that the health condition of the instant examinee is a predetermined health condition.

13. The remote diagnostic aid system according to claim 12, wherein
the predetermined health condition is a health condition designated in advance by one or both of the instant examinee and the attending doctor.

14. The remote diagnostic aid system according to any one of claims 10 to 13, wherein
the stipulation is that the health condition of the instant examinee continues to be a predetermined health condition for a certain period of time.

15. The remote diagnostic aid system according to any one of claims 10 to 14, wherein
the medical expense determination unit determines a payment of an organization such that the payment when the number of examinees associated with the organization is a predetermined number or greater becomes not higher than the payment when the number of examinees associated with the organization is smaller than the predetermined number.

16. A remote diagnostic aid server communicating with one or more doctor terminals and a plurality of user terminals, the remote diagnostic aid server comprising:

a storage unit which stores available consultation time information including an available consultation time during which a doctor as a user of a respective one of the one or more doctor terminals is available for consultation;
a consultation appointment acceptance unit which receives, from a first user terminal as one of the plurality of user terminals, information including a desired consultation time during which an instant examinee as a user of the first user terminal wants to have a consultation, refers to the available consultation time information to extract an attending doctor who is available for consultation during the desired consultation time, and transmits, to one or both of the first user terminal and an attending doctor terminal as the doctor terminal of the attending doctor, communication establishment information for establishing a communication between the attending doctor terminal and the first user terminal; and
a medical expense determination unit which recognizes a condition of the instant examinee on the basis of medical record information received from the attending doctor terminal or the first user terminal, determines whether the condition of the instant examinee fulfills a current stipulation, determines medical expenses for the instant examinee such that the medical expenses when it is determined that the condition of the instant examinee fulfills the current stipulation become not higher than the medical expenses when it is determined that the condition of the instant examinee fails to fulfill the current stipulation, recognizes information indicating a next stipulation on the basis of the medical record information, and transmits the medical expenses, whether the current stipulation has been fulfilled or not, and the information indicating the next stipulation to the first user terminal.

17. A remote diagnostic aid method performed by a remote diagnostic aid server, the remote diagnostic aid server communicating with a plurality of user terminals and one or more doctor terminals, and including a storage unit which stores available consultation time information including an available consultation time during which a doctor as a user of a respective one of the one or more doctor terminals is available for consultation, the method comprising:

a step of receiving, from a first user terminal as one of the plurality of user terminals, information including a desired consultation time during which an instant examinee as a user of the first user terminal wants to have a consultation;

a step of referring to the available consultation time information to extract an attending doctor who is available for consultation during the desired consultation time;

a step of transmitting, to one or both of the first user terminal and an attending doctor terminal as the doctor terminal of the attending doctor, communication establishment information for establishing a communication between the attending doctor terminal and the first user terminal;

a step of recognizing a condition of the instant examinee on the basis of medical record information received from the attending doctor terminal or the first user terminal;

a step of determining whether the condition of the instant examinee fulfills a current stipulation;

a step of determining medical expenses for the instant examinee such that the medical expenses when it is determined that the condition of the instant examinee fulfills the current stipulation become not higher than the medical expenses when it is determined that the condition of the instant examinee fails to fulfill the current stipulation; and

a step of recognizing information indicating a next stipulation on the basis of the medical record information, and transmitting the medical expenses, whether the current stipulation has been fulfilled or not, and the information indicating the next stipulation to the first user terminal.

18. A remote diagnostic aid system being a network system including a remote diagnostic aid server and one or more doctor terminals communicating with a plurality of user terminals, the remote diagnostic aid system comprising:

a storage unit which stores available consultation time information including an available consultation time during which a doctor as a user of a respective one of the one or more doctor terminals is available for consultation;

a consultation appointment acceptance unit which receives, from a first user terminal as one of the plurality of user terminals, information including a desired consultation time during which an instant examinee as a user of the first user terminal wants to have a consultation, refers to the available consultation time information to extract an attending doctor who is available for consultation during the desired consultation time, and transmits, to one or both of the first user terminal and an attending doctor terminal as the doctor terminal of the attending doctor, communication establishment information for establishing a communication between the attending doctor terminal and the first user terminal;

a remote diagnostic aid unit which outputs examinee's statement information, received from the first user terminal with which the communication has been established on the basis of the communication establishment information, to the attending doctor terminal, and transmits doctor's statement information, input from the attending doctor terminal, to the first user terminal; and

a medical expense determination unit which receives medical record information on the instant examinee in a remote diagnosis this time from the attending doctor terminal or the first user terminal, recognizes a next target action of the instant examinee on the basis of the received medical record information, transmits the next target action to the first user terminal, recognizes an adherence level of the instant examinee to a current target action on the basis of the medical record information, and determines medical expenses for the instant examinee in the remote diagnosis this time such that the medical expenses when the adherence level is not lower than a predetermined adherence level become not higher than the medical expenses when the adherence level is lower than the predetermined adherence level.

19. The remote diagnostic aid system according to claim 18, wherein the target action includes at least one of a target action related to eating or drinking, a target action related to exercise, and a target action related to medical equipment.

20. The remote diagnostic aid system according to claim 18 or 19, wherein the medical expense determination unit is configured to transmit the next target action and an importance level of the next target action to the first user terminal, and determine the medical expenses in the remote diagnosis this time in accordance with the current target action, an adherence level to the current target action, and an importance level of the current target action.

21. The remote diagnostic aid system according to any one of claims 18 to 20, wherein the medical expense determination unit is configured to

recognize points this time on the basis of an adherence level to a current target action, and

determine the medical expenses by applying a discount in a case where a stipulation that a state in which the points this time or cumulative points are not smaller than a predetermined threshold value has continued for a certain period of time is fulfilled.

22. The remote diagnostic aid system according to any one of claims 18 to 21, wherein

the medical expense determination unit is configured to determine the medical expenses by applying a discount in a case where a stipulation that a period in which the adherence level to the current target action is not lower than a predetermined threshold level has continued for a certain length of time is fulfilled.

23. A remote diagnostic aid server communicating with a plurality of user terminals and one or more doctor terminals, the remote diagnostic aid server comprising:

a storage unit which stores available consultation time information including an available consultation time during which a doctor as a user of a respective one of the one or more doctor terminals is available for consultation;

a consultation appointment acceptance unit which receives, from a first user terminal as one of the plurality of user terminals, information including a desired consultation time during which an instant examinee as a user of the first user terminal wants to have a consultation, refers to the available consultation time information to extract an attending doctor who is available for consultation during the desired consultation time, and transmits, to one or both of the first user terminal and an attending doctor terminal as the doctor terminal of the attending doctor, communication establishment information for establishing a communication between the attending doctor terminal and the first user terminal; and

a medical expense determination unit which receives medical record information on the instant examinee in a remote diagnosis this time from the attending doctor terminal or the first user terminal, recognizes a next target action of the instant examinee on the basis of the received medical record information, transmits the next target action to the first user terminal, recognizes an adherence level of the instant examinee to a current target action on the basis of the medical record information, and determines medical expenses for the instant examinee in the remote diagnosis this time such that the medical expenses when the adherence level is not lower than a predetermined adherence level become not higher than the medical expenses when the adherence level is lower than the predetermined adherence level.

24. A remote diagnostic aid method performed by a remote diagnostic aid server, the remote diagnostic aid server communicating with a plurality of user terminals and one or more doctor terminals, and including a storage unit which stores available consultation time information including an available consultation time during which a doctor as a user of a respective one of the one or more doctor terminals is available for consultation, the method comprising:

a step of receiving, from a first user terminal as one of the plurality of user terminals, information including a desired consultation time during which an instant examinee as a user of the first user terminal wants to have a consultation;

a step of referring to the available consultation time information to extract an attending doctor who is available for consultation during the desired consultation time;

a step of transmitting, to one or both of the first user terminal and an attending doctor terminal as the doctor terminal of the attending doctor, communication establishment information for establishing a communication between the attending doctor terminal and the first user terminal;

a step of receiving medical record information on the instant examinee in a remote diagnosis this time from the attending doctor terminal or the first user terminal;

a step of recognizing a next target action of the instant examinee on the basis of the received medical record information;

a step of transmitting the next target action to the first user terminal;

a step of recognizing an adherence level of the instant examinee to a current target action on the basis of the medical record information; and

a step of determining medical expenses for the instant examinee in the remote diagnosis this time such that the medical expenses when the adherence level is not lower than a predetermined adherence level become not higher than the medical expenses when the adherence level is lower than the predetermined adherence level.

# FIG.1

**REMOTE DIAGNOSTIC AID SERVER** ~1

**SERVER CONTROL UNIT** ~11
- CONSULTATION APPOINTMENT ACCEPTANCE UNIT ~111
- DIAGNOSTIC AID INFORMATION REGISTRATION UNIT ~112
- EXAMINATION INFORMATION REGISTRATION UNIT ~113
- MEDICAL EXPENSE DETERMINATION UNIT ~114
- ORGANIZATION'S PAYMENT DETERMINATION UNIT ~115
- DATA TRANSMISSION UNIT ~116
- SERVER COMMUNICATION UNIT ~15

**SERVER STORAGE UNIT** ~12
- EXAMINEE LIST ~121
- DOCTOR LIST ~122
- ORGANIZATION LIST ~123
- DIAGNOSTIC AID INFORMATION LIST ~124
- EXAMINATION INFORMATION LIST ~125
- APPOINTMENT LIST ~126
- AVAILABLE EXAMINATION TIME INFORMATION LIST ~127
- TARGETS LIST ~128
- CONDITION LOG ~129

**DOCTOR TERMINAL** ~2
- DOCTOR TERMINAL CONTROL UNIT ~21
- DOCTOR TERMINAL STORAGE UNIT ~22
  - DOCTOR ID ~221
  - COMMUNICATION ESTABLISHMENT INFORMATION ~222
- DOCTOR TERMINAL OUTPUT UNIT ~23
- DOCTOR TERMINAL INPUT UNIT ~24
- DOCTOR TERMINAL COMMUNICATION UNIT ~25

D

NETWORK

P   S

W   UP

**ORGANIZATION TERMINAL** ~4
- ORGANIZATION TERMINAL CONTROL UNIT ~41
- ORGANIZATION TERMINAL STORAGE UNIT ~42
  - ORGANIZATION ID ~421
- ORGANIZATION TERMINAL OUTPUT UNIT ~43
- ORGANIZATION TERMINAL INPUT UNIT ~44
- ORGANIZATION TERMINAL COMMUNICATION UNIT ~45

**FIRST USER TERMINAL** ~3
- FIRST USER TERMINAL CONTROL UNIT ~31
- FIRST USER TERMINAL STORAGE UNIT ~32
  - EXAMINEE ID ~321
  - COMMUNICATION ESTABLISHMENT INFORMATION ~322
- FIRST USER TERMINAL OUTPUT UNIT ~33
- FIRST USER TERMINAL INPUT UNIT ~34
- FIRST USER TERMINAL COMMUNICATION UNIT ~35

**SECOND USER TERMINAL** ~5
- SECOND USER TERMINAL CONTROL UNIT ~51
- SECOND USER TERMINAL STORAGE UNIT ~52
- SECOND USER TERMINAL OUTPUT UNIT ~53
- SECOND USER TERMINAL INPUT UNIT ~54
- SECOND USER TERMINAL COMMUNICATION UNIT ~55

## FIG.2A

| EXAMINEE ID | EXAMINEE NAME | GENDER | AGE | DISEASE NAME | MEMBER NUMBER | CONTACT ADDRESS | COPAYMENT RATE | SETTLEMENT INFORMATION |
|---|---|---|---|---|---|---|---|---|
| 1 | BATSUO MARUTA | MALE | 42 | HYPERTENSION | 1010001 | maruta@aaa.bbb.ccc.co.jp | 30% | 0012-0123-1234-2345/03/18 |
| 2 | KAKUKO SANKAKUYAMA | FEMALE | 38 | DIABETES | 1020002 | sankakuyama@ddd.bbb.ccc.co.jp | 20% | 1234-5678-9012-3456/04/19 |
| 3 | MARUMI BATSUGAWA | FEMALE | 46 | HYPERPIESIA | 1010003 | batsugawa@ddd.bbb.ccc.co.jp | 30% | 9876-5432-1098-7654/10/20 |

## FIG.2B

| DOCTOR ID | DOCTOR NAME | GENDER | SPECIALTY | CONTACT ADDRESS | REMUNERATION DESTINATION INFORMATION |
|---|---|---|---|---|---|
| 11 | BATSUYUKI MARUISHI | MALE | HYPERTENSION, DIABETES, HYPERLIPIDEMIA | maruishi@mmddccrr.co.jp | xxxxxxx |
| 12 | KAKUTA SANKAKUGAWA | MALE | HYPERTENSION, HYPERLIPIDEMIA | sankakugawa@mmddccrr.co.jp | yyyyyyy |
| 13 | MARUKO BATSUYAMA | FEMALE | DIABETES, HYPERLIPIDEMIA | batsuyama@mmddccrr.co.jp | zzzzzzz |

## FIG.2C

| ORGANIZATION ID | ORGANIZATION NAME | SETTLEMENT INFORMATION | SYSTEM UTILIZATION START DATE |
|---|---|---|---|
| 101 | COMPANY A | abcdefg | 2015/12/15 |
| 102 | COMPANY B | hijklmn | 2015/12/20 |

## FIG.2D

| EXAMINEE ID | REGISTRATION DATE AND TIME | BLOOD PRESSURE | TEMPERATURE | WEIGHT | RECENT ILLNESSES | MEDICINES TAKEN RECENTLY | MESSAGE |
|---|---|---|---|---|---|---|---|
| 3 | 2016/4/12 11:30 | 131~75 | 36.4 | 70.1 | NONE | NONE | FEELING BETTER RECENTLY. |
| 1 | 2016/4/19 15:00 | 150~75 | 36.1 | 65.4 | COLD | COLD REMEDY, HYPERTENSION PILL | OCCASIONALLY HAVING PAIN IN THE HEART. |

EP 3 432 254 A1

## FIG.2E

125

| EXAMINEE ID | CONSULTATION DATE AND TIME | SYMPTOMS AND DIAGNOSIS | PRESCRIPTIONS | DOCTOR ID | MEDICAL REMUNERATION POINTS | DOCTOR'S COMMENT |
|---|---|---|---|---|---|---|
| 3 | 2016/1/15 14:00 | HYPERLIPIDEMIA, NO MEDICATION | NONE | 12 | 150 | PLEASE CONTINUE THE ABSTINENCE. |
| 1 | 2016/2/22 13:00 | HYPERTENSION, KEEP THE SAME DOSAGE | MEDICINE 1: 3 CAPSULES PER MEAL FOR 30 DAYS | 11 | 100 | PLEASE SLIGHTLY REDUCE ALCOHOL AND SALT. |
| 2 | 2016/2/23 19:00 | DIABETES, REDUCE THE DOSAGE | MEDICINE 2: 3 CAPSULES PER MEAL FOR 30 DAYS | 13 | 200 | PLEASE MAKE A HABIT OF EXERCISE. |
| 1 | 2016/3/22 18:00 | DIABETES, REDUCE THE DOSAGE | MEDICINE 1: 3 CAPSULES PER MEAL FOR 30 DAYS | 11 | 100 | PLEASE SLIGHTLY REDUCE ALCOHOL AND SALT. |
| 3 | 2016/4/12 11:30 | REMISSION | NONE | 12 | 150 | REMISSION HAS BEEN ACHIEVED. TREATMENT IS FINISHED. PLEASE CONTINUE THE ABSTINENCE, AND GET REGULAR MEDICAL CHECK-UPS. |
| 1 | 2016/4/19 15:00 | HYPERTENSION, REDUCE THE DOSAGE | MEDICINE 1: 2 CAPSULES PER MEAL FOR 30 DAYS | 11 | 100 | PLEASE CONTINUE TO AVOID ALCOHOL AND SALT. PLEASE ACTIVELY FORM AN EXERCISE HABIT. |

1251 1252 1253 1254 1255 1256 1257 1258

## FIG.2F

126

| EXAMINEE ID | DOCTOR ID | APPOINTMENT TIME |
|---|---|---|
| 3 | 12 | 2016/4/12 12:00:00～12:15:00 |
| 1 | 11 | 2016/4/19 15:00:00～15:15:00 |
| 2 | 13 | 2016/4/18 18:00:00～18:10:00 |

1261 1262 1263 1264

## FIG.2G

127

| DOCTOR ID | AVAILABLE CONSULTATION TIME |
|---|---|
| 13 | 2016/4/12 9:00～18:00 |
| 11 | 2016/4/19 15:00～18:00,19:00～21:00 |
| 12 | 2016/4/18 18:00～22:00 |
| 13 | 2016/4/18 19:00～21:00 |

1271 1272

EP 3 432 254 A1

## FIG.2H

128

| TARGET ID (1281) | EXAMINEE ID (1282) | TARGET (1283) | REGISTRATION DATE AND TIME (1284) | IMPORTANCE LEVEL (1285) | ADHERENCE/ ACHIEVEMENT LEVEL (1286) |
|---|---|---|---|---|---|
| 1001 | 1 | HAVE AT LEAST 3 ALCOHOL-FREE DAYS PER WEEK | 2016/4/12 12:00:00 | 100 | 100% |
| 1002 | 1 | DO EXERCISE AT LEAST 3 TIMES A WEEK, CONTINUE EXERCISE FOR 1 HOUR EACH TIME | 2016/4/12 12:00:00 | 120 | 150% |
| 1003 | 1 | WEAR THE WEARABLE SENSOR EVERYDAY | 2016/4/12 12:00:00 | 80 | 50% |
| 1004 | 1 | BLOOD PRESSURE: 160/100 → 150/90 | 2016/4/12 12:00:00 | 150 | 50% |
| 1005 | 1 | WEIGHT: 72 → 70 | 2016/4/12 12:00:00 | 120 | -50% |
| 1006 | 2 | · · · | · · · | · · · | · · · |

## FIG.2I

129

| TARGET ID (1291) | USER INPUT INFORMATION (1292) | SENSING INFORMATION (1293) | DATE AND TIME (1294) |
|---|---|---|---|
| 1001 | HAD 3 ALCOHOL-FREE DAYS | | 2016/4/19 15:00:00 |
| 1002 | DID EXERCISE 5 TIMES, FOR 1 HOUR EACH TIME | | 2016/4/19 15:00:00 |
| 1003 | | WORE EVERYDAY | 2016/4/19 15:00:00 |
| 1004 | | BLOOD PRESSURE: 155/95 | 2016/4/19 15:00:00 |
| 1005 | WEIGHT: 73 | | 2016/4/19 15:00:00 |
| 1006 | · · · | · · · | · · · |

## FIG.3

EP 3 432 254 A1

# FIG.4

**REMOTE DIAGNOSTIC AID SERVER** ⌐1

**DOCTOR TERMINAL** ⌐2

**FIRST USER TERMINAL** ⌐3

STEP120 — AVAILABLE CONSULTATION TIME INPUT? — NO / YES

STEP121 — TRANSMIT THE AVAILABLE CONSULTATION TIME INFORMATION

STEP110 — AVAILABLE CONSULTATION TIME INFORMATION RECEIVED? — NO / YES

STEP111 — STORE THE AVAILABLE CONSULTATION TIME INFORMATION

STEP112 — APPOINTMENT REQUEST INFORMATION RECEIVED? — NO / YES

STEP113 — EXTRACT ATTENDING DOCTOR

STEP114 — ADD, TO APPOINTMENT LIST, INSTANT EXAMINEE, ATTENDING DOCTOR, AND APPOINTMENT TIME

STEP115 — TRANSMIT APPOINTMENT ACCEPTANCE INFORMATION

STEP116 — TRANSMIT ASSIGNED APPOINTMENT INFORMATION

STEP130 — APPOINTMENT REQUEST MANIPULATION DETECTED? — NO / YES

STEP131 — TRANSMIT APPOINTMENT REQUEST INFORMATION

STEP122 — ASSIGNED APPOINTMENT INFORMATION RECEIVED? — NO / YES

STEP123 — OUTPUT THE APPOINTMENT TIME AND THE EXAMINEE INFORMATION INCLUDED IN THE ASSIGNED APPOINTMENT INFORMATION

STEP124 — STORE THE COMMUNICATION ESTABLISHMENT INFORMATION INCLUDED IN THE ASSIGNED APPOINTMENT INFORMATION

STEP132 — APPOINTMENT ACCEPTANCE INFORMATION RECEIVED? — NO / YES

STEP133 — OUTPUT THE NOTIFICATION THAT THE APPOINTMENT HAS BEEN ACCEPTED AND THE DOCTOR INFORMATION INCLUDED IN THE APPOINTMENT ACCEPTANCE INFORMATION

STEP134 — STORE THE COMMUNICATION ESTABLISHMENT INFORMATION INCLUDED IN THE APPOINTMENT ACCEPTANCE INFORMATION

END     END     END

EP 3 432 254 A1

# FIG.5

REMOTE DIAGNOSTIC AID SERVER $\sim$ 1    DOCTOR TERMINAL $\sim$ 2    FIRST USER TERMINAL $\sim$ 3    WEARABLE SENSOR $\sim$ S

STEP230
NO — DIAGNOSTIC AID INFORMATION INPUT?

YES    STEP231
TRANSMIT THE DIAGNOSTIC AID INFORMATION

STEP210
NO — DIAGNOSTIC AID INFORMATION RECEIVED?

YES    STEP211
REGISTER THE DIAGNOSTIC AID INFORMATION

STEP232
TRANSMIT SENSING INFORMATION

STEP212
NO — SENSING INFORMATION RECEIVED?

YES    STEP213
REGISTER THE SENSING INFORMATION

STEP214
TRANSMIT THE DIAGNOSTIC AID INFORMATION AND THE SENSING INFORMATION

STEP220
DIAGNOSTIC AID INFORMATION AND SENSING INFORMATION RECEIVED? — NO

YES    STEP221
OUTPUT THE DIAGNOSTIC AID INFORMATION AND THE SENSING INFORMATION

END    END    END    END

EP 3 432 254 A1

FIG.6A

FIG.6B

Figure 6B — Flowchart

(1)

EXAMINATION INFORMATION RECEIVED? — STEP310
NO (loop back)
YES
REGISTER THE EXAMINATION INFORMATION — STEP311
TRANSMIT CANDIDATE FOR TARGET — STEP312
NEXT TARGET RECEIVED? — STEP313
NO (loop back)
YES
REGISTER THE NEXT TARGET — STEP314
END

(2)

CREATE EXAMINATION INFORMATION — STEP328
TRANSMIT THE EXAMINATION INFORMATION — STEP329
END

(3)

CANDIDATE FOR NEXT TARGET RECEIVED? — STEP338
NO (loop back)
YES
DISPLAY A SCREEN FOR CONFIRMATION OF NEXT TARGET — STEP339
TRANSMIT THE INPUT NEXT TARGET — STEP340
END

# FIG.7A

**REMOTE MEDICAL AID SERVER** 1

STEP401
MEDICAL EXPENSE DETERMINATION PROCESSING

STEP410
DETERMINE COPAYMENT

STEP411
TRANSMIT THE PAYMENT OF MEDICAL EXPENSES, EXAMINATION INFORMATION, AND TARGETS INFORMATION

STEP412
SETTLEMENT PROCESSING

STEP413
DETERMINE MEDICAL REMUNERATION

STEP414
TRANSMIT THE MEDICAL REMUNERATION DATA

STEP415
SETTLE THE REMUNERATION PAYMENT

END

**DOCTOR TERMINAL** 2

STEP420
MEDICAL REMUNERATION DATA RECEIVED? — NO

YES

STEP421
OUTPUT THE MEDICAL REMUNERATION DATA

END

**FIRST USER TERMINAL** 3

STEP430
PAYMENT OF MEDICAL EXPENSES, EXAMINATION INFORMATION, AND TARGETS INFORMATION RECEIVED? — NO

YES

STEP431
OUTPUT THE PAYMENT OF MEDICAL EXPENSES, EXAMINATION INFORMATION, AND TARGETS INFORMATION

END

# FIG.7B

REMOTE MEDICAL AID SERVER

STEP4011

RECOGNIZE MEDICAL REMUNERATION POINTS

STEP4012

RECOGNIZE MEDICAL EXPENSES IN
CASE OF FACE-TO-FACE DIAGNOSIS

STEP4013

RECOGNIZE MEDICAL EXPENSES FOR REMOTE DIAGNOSIS

STEP4014

RECOGNIZE POINTS THIS TIME AND CUMULATIVE POINTS

STEP4015

APPLY DISCOUNT ACCORDING TO THE POINTS

END

# FIG.8

| | | | |
|---|---|---|---|
| EXAMINEE NAME | BATSUO MARUTA | | MR. |
| EXAMINEE ID | 1 | ATTENDING DOCTOR NAME | BATSUYUKI MARUISHI |
| CONSULTATION DATE AND TIME | 2016/4/19 15:00 | DOCTOR ID | 11 |

| | |
|---|---|
| SYMPTOMS AND DIAGNOSIS | HYPERTENSION, REDUCE THE DOSAGE |

| PRESCRIPTIONS | MEDICINE 1: 2 CAPSULES AFTER EACH MEAL FOR 30 DAYS | TRANSMIT THE PRESCRIPTIONS TO PHARMACY |
|---|---|---|

PLEASE CONTINUE TO AVOID ALCOHOL AND SALT.
PLEASE ACTIVELY FORM AN EXERCISE HABIT.

| CURRENT TARGET | HAVE AT LEAST 3 ALCOHOL-FREE DAYS PER WEEK | DO EXERCISE AT LEAST 3 TIMES A WEEK, FOR 1 HOUR EACH TIME | WEAR THE WEARABLE SENSOR EVERYDAY | BLOOD PRESSURE : 150/90 | WEIGHT: 72 → 70 OR LESS |
|---|---|---|---|---|---|
| IMPORTANCE LEVEL | 100 | 120 | 80 | 150 | 120 |
| ADHERENCE/ ACHIEVEMENT LEVEL | 100% | 150% | 50% | 50% | -50% |
| POINTS THIS TIME | 335 PT + 5% DISCOUNT ACHIEVED! | CUMULATIVE POINTS | | 1050 PT + 5% DISCOUNT ACHIEVED! | |
| NEXT TARGET | HAVE AT LEAST 3 ALCOHOL -FREE DAYS PER WEEK | EAT RAMEN NOODLES NOT MORE THAN TWICE A WEEK | WEAR THE WEARABLE SENSOR EVERYDAY | BLOOD PRESSURE: 150/90 | WEIGHT: 73→ 72 OR LESS |
| IMPORTANCE LEVEL | 100 | 80 | 80 | 150 | 120 |
| COPAYMENT THIS TIME | | | 300 YEN | MAKE THE NEXT APPOINTMENT | |

EP 3 432 254 A1

# FIG.9

REMOTE MEDICAL AID SERVER ⌐1

STEP510

RECOGNIZE ORGANIZATION'S STANDARD PAYMENT OF
MEDICAL EXPENSES IN CASE OF FACE-TO-FACE DIAGNOSIS

STEP511

RECOGNIZE ORGANIZATION'S PAYMENT OF
MEDICAL EXPENSES IN CASE OF REMOTE DIAGNOSIS

STEP512

DETERMINE A SYSTEM UTILIZATION COST

STEP513

IS THE NUMBER OF USERS PER UNIT PERIOD BELONGING
TO THE ORGANIZATION A PREDETERMINED NUMBER OR MORE? — NO

YES

STEP514

REDUCE THE ORGANIZATION'S PAYMENT

STEP515

SETTLEMENT

END

# FIG.10

| ORGANIZATION TERMINAL ⌐4 | REMOTE MEDICAL AID SERVER ⌐1 |

**STEP640**
MANIPULATION INPUT? — NO
↓ YES

**STEP641**
TRANSMIT REQUEST FOR DATA ON ORGANIZATION'S PAYMENT OF MEDICAL EXPENSES

**STEP610**
REQUEST FOR DATA ON ORGANIZATION'S PAYMENT OF MEDICAL EXPENSES RECEIVED? — NO
↓ YES

**STEP611**
FROM THE ORGANIZATION ID, SPECIFY EXAMINEE IDS OF THE EXAMINEES BELONGING TO THE ORGANIZATION

**STEP612**
RECOGNIZE EXAMINATION INFORMATION OF THE SPECIFIED EXAMINEE IDS

**STEP613**
CALCULATE ORGANIZATION'S PAYMENT OF MEDICAL EXPENSES

**STEP614**
RECOGNIZE ORGANIZATION'S STANDARD PAYMENT OF MEDICAL EXPENSES

**STEP615**
CALCULATE A SYSTEM UTILIZATION COST

**STEP616**
CREATE TIME-SERIES DATA ON ORGANIZATION'S PAYMENT OF MEDICAL EXPENSES

**STEP617**
TRANSMIT THE DATA ON ORGANIZATION'S PAYMENT OF MEDICAL EXPENSES

**STEP642**
DATA ON ORGANIZATION'S PAYMENT OF MEDICAL EXPENSES RECEIVED? — NO
↓ YES

**STEP643**
OUTPUT THE DATA ON ORGANIZATION'S PAYMENT OF MEDICAL EXPENSES

END                    END

46

## FIG.11A

AMOUNT OF MONEY

SYSTEM UTILIZATION START DATE          PERIOD

## FIG.11B

t1

BLOOD PRESSURE

DAILY DOSE OF MEDICINE

FIRST CONSULTATION DATE          PERIOD

## FIG.11C

t2

BLOOD PRESSURE

FIRST CONSULTATION DATE          PERIOD

# FIG.12

```
          ┌─3                                    ┌─1
┌──────────────────────────┐          ┌──────────────────────────┐
│    FIRST USER TERMINAL    │          │  REMOTE MEDICAL AID SERVER │
└──────────────────────────┘          └──────────────────────────┘
```

**STEP730**

NO — MANIPULATION INPUT?

YES — **STEP731**

TRANSMIT REQUEST FOR FIRST HEALTH CONDITION DATA

**STEP710**

REQUEST FOR FIRST HEALTH CONDITION DATA RECEIVED? — NO

YES

**STEP711**

ON THE BASIS OF THE EXAMINEE ID, RECOGNIZE DIAGNOSTIC AID INFORMATION AND EXAMINATION INFORMATION

**STEP712**

RECOGNIZE TIME-SERIES HEALTH CONDITIONS OF THE INSTANT EXAMINEE

**STEP713**

CREATE FIRST HEALTH CONDITION DATA

**STEP714**

TRANSMIT THE FIRST HEALTH CONDITION DATA

**STEP732**

NO — FIRST HEALTH CONDITION DATA RECEIVED?

YES — **STEP733**

OUTPUT THE FIRST HEALTH CONDITION DATA

END

END

# FIG.13

| SECOND USER TERMINAL ⌐5 | REMOTE MEDICAL AID SERVER ⌐1 |

**STEP850**
NO — DISEASE NAME INPUT?
YES

**STEP851**
TRANSMIT REQUEST FOR SECOND HEALTH CONDITION DATA

**STEP810**
REQUEST FOR SECOND HEALTH CONDITION DATA RECEIVED? — NO
YES

**STEP811**
RECOGNIZE THE EXAMINEE IDS ASSOCIATED WITH THE DISEASE NAME

**STEP812**
ON THE BASIS OF THE EXAMINEE IDS, RECOGNIZE DIAGNOSTIC AID INFORMATION AND EXAMINATION INFORMATION

**STEP813**
RECOGNIZE TIME-SERIES HEALTH CONDITIONS OF THE RESPECTIVE EXAMINEES

**STEP814**
CREATE SECOND HEALTH CONDITION DATA

**STEP815**
TRANSMIT THE SECOND HEALTH CONDITION DATA

**STEP852**
NO — SECOND HEALTH CONDITION DATA RECEIVED?
YES

**STEP853**
OUTPUT THE SECOND HEALTH CONDITION DATA

( END )    ( END )

# FIG.14

130

| EXAMINEE ID | STANDARD PAYMENT BY INSURANCE ORGANIZATION |
|:---:|:---:|
| 1 | 10000 |
| 2 | 8000 |
| 3 | 12000 |

1301      1302

# FIG.15

EP 3 432 254 A1

# FIG.16

REMOTE MEDICAL AID SERVER

STEP1010

DETERMINE INSURANCE
ORGANIZATION'S PAYMENT

STEP1011

DETERMINE SYSTEM UTILIZATION COST

STEP1012

MAKE SETTLEMENT OF THE
SYSTEM UTILIZATION COST

END

## INTERNATIONAL SEARCH REPORT

| | International application No. |
|---|---|
| | PCT/JP2017/010791 |

**A. CLASSIFICATION OF SUBJECT MATTER**
*G06Q50/22*(2012.01)i, *G06Q40/08*(2012.01)i

According to International Patent Classification (IPC) or to both national classification and IPC

**B. FIELDS SEARCHED**

Minimum documentation searched (classification system followed by classification symbols)
G06Q10/00-99/00

Documentation searched other than minimum documentation to the extent that such documents are included in the fields searched
Jitsuyo Shinan Koho          1922-1996   Jitsuyo Shinan Toroku Koho   1996-2017
Kokai Jitsuyo Shinan Koho    1971-2017   Toroku Jitsuyo Shinan Koho   1994-2017

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| Y<br>A | JP 2015-530886 A  (Boucher Ryan),<br>29 October 2015 (29.10.2015),<br>paragraphs [0046] to [0229]; fig. 3<br>& WO 2014/004905 A1<br>paragraphs [0066] to [0316]; fig. 3 | 10-17<br>1-9,18-24 |
| Y | WO 2006/098298 A1  (Matsushita Electric Industrial Co., Ltd.),<br>21 September 2006 (21.09.2006),<br>paragraph [0008]<br>& US 2008/0065413 A1<br>paragraph [0008] | 10-17 |
| Y | JP 2015-69531 A  (LSI Medience Corp.),<br>13 April 2015 (13.04.2015),<br>paragraph [0007]<br>(Family: none) | 10-17 |

☒  Further documents are listed in the continuation of Box C.          ☐  See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 26 May 2017 (26.05.17) | 06 June 2017 (06.06.17) |

| Name and mailing address of the ISA/<br>Japan Patent Office<br>3-4-3,Kasumigaseki,Chiyoda-ku,<br>Tokyo 100-8915,Japan | Authorized officer<br><br>Telephone No. |
|---|---|

Form PCT/ISA/210 (second sheet) (January 2015)

**INTERNATIONAL SEARCH REPORT**

International application No.

PCT/JP2017/010791

C (Continuation).   DOCUMENTS CONSIDERED TO BE RELEVANT

| Category* | Citation of document, with indication, where appropriate, of the relevant passages | Relevant to claim No. |
|---|---|---|
| Y | JP 2010-503122 A  (American Well Inc.), 28 January 2010 (28.01.2010), paragraph [0090] & WO 2008/030855 A1 page 30, lines 7 to 18 | 10-17 |
| P,X | Hideyuki AKASHI, "Iryohi Sakugen o Ninau Enkaku Shinryo", a monthly journal of medical imaging and information, 2016.04, vol.48, no.4, pages 16 to 19, ISSN 1346-1354, particularly, pages 17 to 18 | 1,6-9 |
| A | WO 2002/17171 A1  (Arkray, Inc.), 28 February 2002 (28.02.2002), entire text; all drawings & US 2004/0117200 A1 entire text; all drawings & CN 1470032 A | 1-24 |
| A | JP 2002-117152 A  (Nippon Telegraph and Telephone Corp.), 19 April 2002 (19.04.2002), entire text; all drawings (Family: none) | 1-24 |
| A | JP 11-347003 A  (Toshiba Corp.), 21 December 1999 (21.12.1999), entire text; all drawings (Family: none) | 1-24 |
| A | JP 2015-90572 A  (Masaaki NAGANO), 11 May 2015 (11.05.2015), entire text; all drawings (Family: none) | 1-24 |
| A | US 2014/0278491 A1  (WEISS David I.), 18 September 2014 (18.09.2014), entire text; all drawings (Family: none) | 1-24 |
| A | JP 2007-257565 A  (Hitachi, Ltd.), 04 October 2007 (04.10.2007), paragraphs [0018] to [0024], [0042] to [0048]; fig. 4 & US 2007/0226007 A1 paragraphs [0042] to [0048], [0066] to [0072]; fig. 4 | 2-5 |
| A | JP 2012-164244 A  (KDDI Corp.), 30 August 2012 (30.08.2012), paragraphs [0008], [0016] to [0025] (Family: none) | 4-5 |

Form PCT/ISA/210 (continuation of second sheet) (January 2015)

**EP 3 432 254 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2015090572 A